# EUROPEAN PATENT SPECIFICATION

(11) **EP 1 011 433 B1**
(45) Date of publication and mention of the grant of the patent: **25.02.2009**
(21) Application number: 98908791.1
(22) Date of filing: 06.03.1998
(51) Int. Cl.: A61B 5/021, A61B 5/04, A61B 5/00, A61J 7/04

(54) **METHOD FOR REAL-TIME MONITORING AND MANAGEMENT OF PATIENTS' HEALTH STATUS AND MEDICAL TREATMENT REGIMENS**
VERFAHREN ZUR ECHTZEIT-ÜBERWACHUNG DES GESUNDHEITSZUSTANDES UND DES BEHANDLUNGSPLANS VON PATIENTEN
PROCEDE POUR LA SURVEILLANCE ET LA GESTION TEMPS REEL DE L'ETAT DE SANTE ET DES SCHEMAS POSOLOGIQUES DE TRAITEMENTS MEDICAUX DES PATIENTS

(30) Priority: 07.03.1997 US 40128 P; 18.04.1997 US 44472 P; 01.07.1997 US 51389 P; 08.09.1997 US 924917; 22.10.1997 US 955952; 22.12.1997 US 68473 P
(43) Date of publication of application: 28.06.2000
(73) Proprietor: Informedix, Inc., Rockville, MD 20852 (US)
(72) Inventor: KEHR, Bruce, A., Potomac, MD 20854 (US); BENSON, Robert, H., Bethesda, MD 20817 (US); SOHN, Evan, New York, NY 10003 (US); STARNES, James, E., Crownsville, MD 21032 (US); MAURER, David, Randolph, NJ 07869 (US); STOWELL, Davin, New York, NY 10003 (US); CHAPMAN, Dean, Brooklyn, NY 11217 (US); FARRAGE, David, Weehawken, NJ 07087-7013 (US); BAUMEL, Irwin, D., Delray Beach, FL 33446 (US); STEMPLER, David, S., Potomac, MD 20854 (US)
(74) Representative: Müller-Boré & Partner Patentanwälte
(86) International application number: PCT/US1998/003933
(87) International publication number: WO 1998/038909

(56) References cited:
- EP-A- 0 526 166
- WO-A-89/05116
- US-A- 4 958 645
- US-A- 5 200 891
- US-A- 5 596 994
- US-A- 5 710 178
- US-A- 5 710 551
- US-A- 5 722 418

## Description

### FIELD OF THE INVENTION

The present invention relates to a method for performing real-time monitoring of a patient's health status using a medical monitoring device.

### BACKGROUND OF THE INVENTION

The prior art discloses a number of electronic devices that assist with the administration of prescribed medication and monitor the medical treatment progress. Medication monitoring devices such as those disclosed in U.S. Patent Nos . 5,200,891 and 5,642,731 provide a number of functions for facilitating patient adherence to prescribed therapies, and for facilitating cross-correlation of compliance data and clinical information about the patient.

Such devices have a plurality of medication compartments, a microprocessor with associated circuitry for providing timing signals, signals and display messages and for reading inputs from buttons that convey programming and operating information. They rely on programmed schedules for providing audible and/or visual alert signals at the scheduled times for taking certain medications and indicate the specific compartment from which the particular medication is to be taken, and quantity to be taken. The device of U.S. Patent No. 5,642,731 is also capable of collecting contemporaneous data concerning the patient's adherence to the medication schedule, the progression of the medical condition(s) being treated, symptoms and side effects the patient is experiencing and other information pertinent to monitoring and treating the patient's medical condition(s).

However, the prior art suffers from a number of inherent disadvantages that have been solved by the present invention. The prior art devices contain complex operating systems rendering them difficult to understand how to operate and extract medical and educational information, and lack the functionality for easily presenting patient queries and obtaining patient responses, and easily communicating with remote devices. The operating systems of the prior art devices are also inadequate in their ability to provide for differentiation of operating sequences pertinent to specific medications and treatment progress, further complicating their operation and monitoring of treatment regimens.

Further, the prior art devices lack the ability to gather contemporaneous data reflecting treatment progress, medical treatment regimen compliance, and patient health status through either a simple patient query interface, or monitors physically attached to the patient that monitor physiologic, cellular, molecular, endocrine and/or metabolic events. The prior art devices also lack the ability to automatically adjust a treatment regimen based on an algorithm within the device that analyzes the contemporaneous data collected, and automatically adjusts a treatment regimen accordingly. The prior art devices also lack the ability to provide positive and negative feedback, and warnings to the patient, based upon the contemporaneous data collected. The prior art devices also suffer from a difficulty in giving repetitive reinforcing information on a per-medication basis, to encourage better compliance with the entire medical treatment regimen.

Further, the prior art devices fail to provide for automatic communication and transfer of data and information between the device and remote devices. In this regard, the prior art devices also lack the ability to provide for immediate transmission of the contemporaneous data reflecting the patient's treatment progress. Immediate transmission would enable a healthcare provider to perform a real-time evaluation of the contemporaneous data and adjust the patient's treatment regimen accordingly (the adjustment may range from simply revising medication schedules to requesting that the patient schedule an appointment with a physician, or even requiring that the patient proceed to a hospital emergency room) .

The prior art devices further lack functionality for intensive, real-time monitoring of outpatients, which would provide the ability to monitor outpatients at an intensity relatively equal to that of monitoring patient's in a hospital.

With regard to the identification of the medications stored in the device, the prior art lacks the ability to provide for easy identification of the medication contained in each compartment. Additionally, the prior art devices include fixed volume medication storage capacities that limit the device's flexibility and portability, and are unable to easily provide the patient with reminders as to when to reload specific medication (s), which medication compartments to reload, how many to reload, and what the medication looks like.

EP 0 526 166 A2 refers to a method of monitoring a patient's medication compliance. I involves utilizing an automatic compliance monitoring device which tracks patient compliance data, along with a wave energy transmitter and power source, all connected to a medicine container. The wave energy transmitter on the medicine container continuously, periodically, or randomly transmits raw or calculated data to the receiver and from there to the computer to compare actual usage with compliance required, to determine compliance results on the display unit to permit compliance monitoring on a monitor at a remote location.

US 5 596 994 A refers to an automated and interactive positive motivation system that allows a physician, counselor or trainer to produce and send a series of motivational messages and/or questions to a client to change or reinforce a specific behavioral problem. The database and program are operated by a computer that at preselected time periods sends the messages and/or questions to the client through use of a variety of transmission means and furthermore selects a platform of behavioral issues that is to be addressed based on a given behavioral stage at a given time of day.

Document WO-A-89/05 116 discloses a method for monitoring the health status of a patient (eg, EKG or respiration) by analysing the monitored resulting data by comparing them to programmable limits, generating alarms and wirelessly transmitting them to a remote device when the detected data exceed the programmable limits, and storing the data when no execution command requires immediate transmission.

US 5 200 891 A refers to a device for monitoring medication of a patient for prompting the patient into certain medication taking schedule and/or certain programming steps and routines.

Thus, according to an aspect, it is a problem to facilitate efficient and improved monitoring and management of medical treatment regimens.

This problem is solved by the method as claimed in claim 1. Preferred embodiments are defined in the dependent claims.

### SUMMARY OF THE INVENTION

The present invention provides a method for performing real time monitoring of a patient's health status using a medical monitoring device. The present invention provides for real-time access to important treatment information, real-time adjustment of treatment regimens and monitoring devices and sequences, and real-time patient/healthcare provider interaction, facilitating efficient and improved monitoring and management of medical treatment regimens. The present invention also provides for the collection of information concerning the treatment of the patient's medical condition(s) to assist the health care professional in monitoring the progression of the patient's medical condition(s) and treatment results. The invention collects contemporaneous information concerning the patient's compliance with the treatment regimen, the progression of the patient's medical condition(s), symptoms and side effects experienced by the patient, physiologic information (i.e., blood pressure or glucose levels), endocrine or cellular functioning (i.e., presence and quantity of circulating or serum polypeptide or steroid hormone levels, hepatic enzymes or other cellular metabolites detectable by a specific assay device containing receptors or antibodies, etc.), and the patient's general health status and quality of life. This information facilitates prompt, accurate, real-time analysis of the patient's progress, resulting in more efficient management of the patient's medical condition(s), health, and improved treatment results. The present invention provides a medical monitoring device that interfaces with a communications cradle or base unit for transferring data to and from remote devices. The present invention also provides for a medical monitoring device that is capable of direct communication to and from remote devices via wireless communication methods commonly known to those of ordinary skill in the art of electronic communication. The information is either immediately communicated to a remote device, or stored in the medical monitoring device for subsequent communication.

In an embodiment, the present invention provides a method for wireless communication with and polling of the medical monitoring device. The wireless communication may be by a cellular telephone link, satellite link, or other radio communication means, commonly known to those of ordinary skill in the art of electronic communications. The wireless communication feature allows the transmission of patient query data to the medical monitoring device, for conducting a health status assessment, and monitoring adverse effects or side effects experienced by the patient, treatment progress, and the patient's general quality of life- The wireless communication feature also facilitates polling of the medical monitoring device or other monitoring devices to retrieve patient response data, physiologic data, hormones, enzymes or other metabolite concentrations, and any other physical, chemical, molecular, endocrine, or metabolic data related to the patient for real-time analysis of the patient's health status and treatment progress.

The wireless communication feature provides improvements over the prior art in that a medical monitoring device with such a feature does not necessarily require a long-term memory storage function (i.e., week or month). The wireless communication feature also facilitates periodic, spontaneous, and or random, data transmission or polling of data, related to the patient's treatment regimen and health status. In addition, the device is capable of transmitting data concerning the patient's condition promptly after a particular data element, sequence of data elements, or combination of data elements is entered by the user. The wireless transmission feature thereby enables an increase in the frequency and intensity of monitoring, up to the capacity for real-time monitoring of outpatients. Further, the wireless communication feature provides for prompt data transfer where the patient's condition warrants prompts attention. The wireless communication feature thereby allows for outpatient treatment of seriously ill patients, to keep them out of hospitals and nursing homes, with more independence, and less health care expense.

### BRIEF DESCRIPTION OF THE DRAWINGS

Fig. 1 illustrates a block diagram of the electronic circuitry of the medical monitoring device.
Figs. 2(a) and 2(b) illustrate a top/side view and a bottom/rear/side view of the medical monitoring device, including the dedicated and soft function keys, display, and infrared communications port.
Fig. 3 illustrates the cradle and the interface between the cradle and medical monitoring device.
Fig. 4 illustrates a flow chart of the general operation of the medical monitoring device, operating system and method.
Fig. 5 illustrates the liquid crystal display, dedicated keys and soft function keys in the passive mode of the medical monitoring device, operating system and method.
Figs. 6(a)-(c) illustrate the liquid crystal display, dedicated keys and soft function keys during a dedicated key operating sequence of the medical monitoring device, operating system and method.
Figs. 7(a)-(e) illustrate the liquid crystal display, dedicated keys and soft function keys during an operating sequence of a scheduled medication alarm operating sequence of the medical monitoring device, operating system and method.
Figs. 8(a)-(b) illustrate the liquid crystal display, dedicated keys and soft function keys during an unscheduled medication alarm operating sequence of the medical monitoring device, operating system and method.
Figs- 9(a)-(b) illustrate the liquid crystal display, dedicated keys and soft function keys during different general signals of the medical monitoring device, operating system and method.
Figs. 10(a)-(b) illustrate the liquid crystal display, dedicated keys and soft function keys during a mode-select function of the medical monitoring device, operating system and method.
Figs. 11 (a)-(c) illustrate the liquid crystal display, dedicated keys and soft function keys during a "Medication Compliance" mode operating sequence of the medical monitoring device, operating system and method.
Figs. 12(a)-(d) illustrate the liquid crystal display, dedicated keys and soft function keys during a "Patient Query" mode operating sequence of the medical monitoring device, operating system and method.
Figs. 13(a)-(h) illustrate the liquid crystal display, dedicated keys and soft function keys during a "Medication Schedule - Program" mode operating sequence of the medical monitoring device, operating system and method.
Figs. 14(a)-(c) illustrate the liquid crystal display, dedicated keys and soft function keys during a "Refill" alarm operating sequence of the medical monitoring device, operating system and method.
Figs. 15 (a) - (b) illustrate the liquid crystal display, dedicated keys and soft function keys during a "refill" mode operating sequence of the medical monitoring device, operating system and method.
Fig. 16 illustrates a removable tray of one embodiment for use with the medical monitoring device, operating system and method.
Fig. 17 illustrates a removable tray of a further embodiment for use with the medical monitoring device, operating system and method.
Fig- 18 illustrates a removable tray of a further embodiment for use with the medical monitoring device, operating system and method.
Fig. 19 illustrates a removable tray storing a pill bottle for use with the medical monitoring device, operating system and method.
Fig- 20 illustrates a removable tray with sealed, labeled medication compartments for use with the medical monitoring device, operating system and method.
Figs. 21 (a) and 21(b) illustrate two views of the medical monitoring device and removable tray with labels on the underside of the device.
Fig. 22 illustrates a removable tray and compartment insert for use with the medical monitoring device, operating system and method.
Fig. 23 illustrates the removable tray and frame or support cover for use with the medical monitoring device, operating system and method.
Fig. 24 illustrates a block diagram of a medical monitoring system of monitors, sensors, delivery devices, and remote devices.

### DETAILED DESCRIPTION OF EXEMPLARY EMBODIMENTS

The present invention provides a method that assists a patient user with the administration of medical treatment regimens, and assists health care providers with providing the most efficient and effective treatment for medical conditions. Similar devices are disclosed in U.S. Patent Nos. 5,200,891 and 5,642,731.

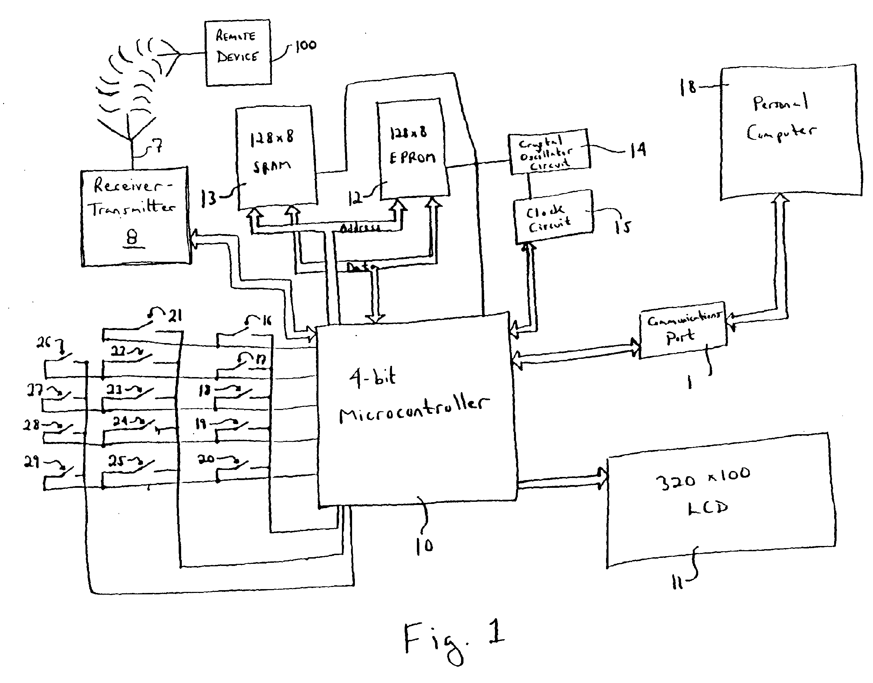

The medical monitoring device illustrated in Fig.1 utilizes a 4-bit microcontroller chip 10 interfaced with a 320x100 pixel liquid crystal display 11 ("LCD") with graphics capabilities. The device operating system is stored in a 128x8 bit external EPROM 12 and the variable data (i.e. medication scheduling data, treatment information, patient query data, and response data) is stored in an external 128x8 bit SRAM 13. The crystal oscillator circuit 14 provides a clock signal to the microcontroller 10, the clock circuit 15 tracks the date and time of day. Five compartment switches 16-20, five dedicated keys 21-25 and four soft function keys 26-29 are connected to inputs of the microcontroller 10 for supplying status signals, function commands and patient query responses to the microcontroller 10 for processing.

The device enables interaction with health care providers through a communication port 17. Communication port 17 comprises an infrared input/output port capable of communicating directly with a personal computer 18. Alternatively, the communication port may comprise a serial port capable of communicating with a modem or directly with a PC.

The health care provider can download information to the device, such as programming data concerning the prescribed medication schedules, programming data concerning the selection and control of audible and/or visible signals (i.e., sounds, music, melodies, tones, colored lights, etc. for communication with impaired patients), information concerning the prescribed medication and medical condition(s) being treated, and patient queries associated with the medical condition being treated. The health care provider can also upload information from the device, such as data concerning the patient's adherence to the prescribed medication schedules and information representing the patient's responses to patient queries.

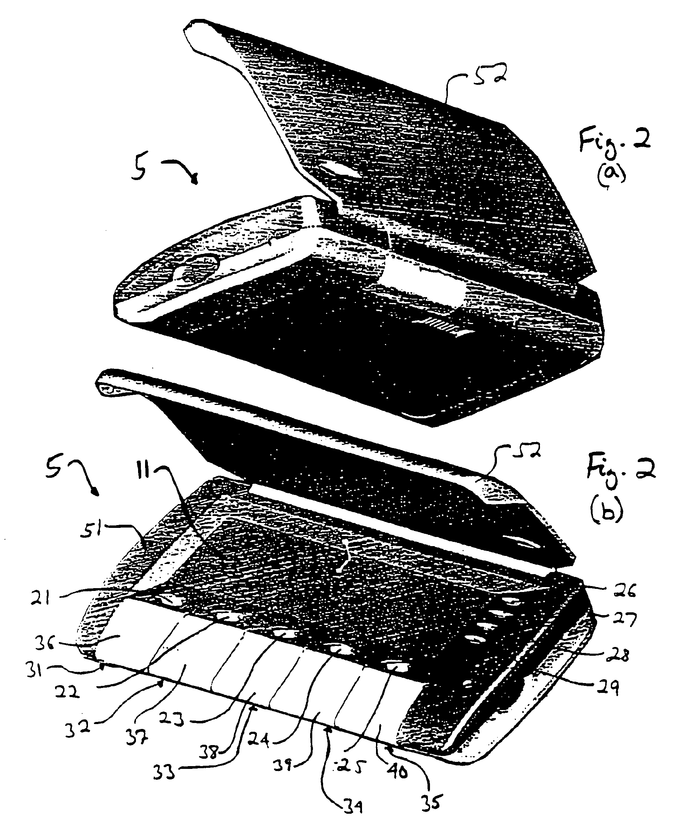

The device itself, as illustrated in Figs. 2(a) and 2(b), has a top face 51. The top face 51 includes openings for the LCD 11, dedicated keys 21-25, soft function keys 26-29 and medication compartments 31-35. The medication compartments 31-35 have respective compartment doors 36-40 covering them. The device also has a hinged top cover 52 for opening and closing the device. The medication compartments 31-35 are housed in a tray that inserts into the bottom of the device. The tray may be of various sizes, and may even be large enough to maintain a pill bottle, a medication inhaler, syringe, or other large medication container. Further, the medication compartments may directly accommodate liquid, injectable, or aerosolized medications.

Compartment switches 16-20 signal the microcontroller 10, indicating when compartment doors are opened and closed. Each of the medication compartments 31-35 and its respective compartment door is associated with a compartment switch that senses the opening and closing of the compartment door. When a particular compartment door is opened, the associated compartment switch signals the microcontroller 10, and the microcontroller processes the signal according to the specific status of the device at that time. For example, as described in detail below, if a compartment door is opened during a scheduled medication alarm, the microcontroller 10 interprets this as an indication that the patient has complied with the prescribed schedule for the particular medication contained in that compartment. The compartment switches thereby facilitate the device's tracking of the patient's adherence to prescribed medication schedules.

The device provides a means for interacting with the patient through a series of operations controlled by the dedicated keys 21-25 and soft function keys 26-29. The dedicated keys 21-25 enable the patient to retrieve information concerning specific medications contained in the device's medication compartments or concerning specific diseases. The soft function keys 26-29 enable the patient to respond to patient queries concerning medication side effects, adherence to medication schedules, treatment progress, health status assessment and patient's quality of life. The soft function keys 26-29 further facilitate other operations, such as a programming mode for modifying device settings and adjusting medication schedules and treatment data, and other types of information capture.

Each of the dedicated keys is associated with the particular medication compartment located adjacent to it. Specifically, dedicated key 21 is associated with medication compartment 31, dedicated key 22 is associated with medication compartment 32, dedicated key 23 is associated with medication compartment 33, dedicated key 24 is associated with medication compartment 34, and dedicated key 25 is associated with medication compartment 35. Each dedicated key controls the display or entry of information related to the specific medication stored in its associated medication compartment or related to the medical condition for which that medication is prescribed.

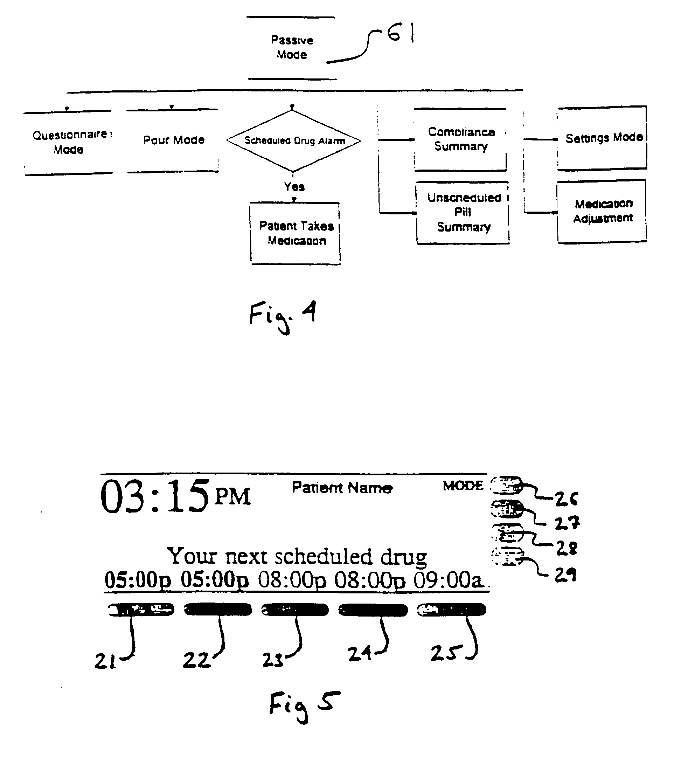

Referring to Figs. 4 and 5, once programmed, the device remains in a passive mode 61 from which one of a plurality of events can occur. In the passive mode 61, the device displays the current time of day, the patient's name and the patient's upcoming medication schedule. Alternatively, the device may also display the date. The display of the patient's drug schedule comprises a display, located directly above each dedicated key, of the next prescribed time that the patient should take the medication contained in the medication compartment associated with that key. For example, as illustrated in Fig. 5, the time of day is 3:15 p.m., and the patients drug schedule entails taking the medication contained in compartments 21 and 22 at 5:00 p.m., compartments 23 and 24 at 8:00 p.m. and compartment 25 at 9:00 am the following morning.

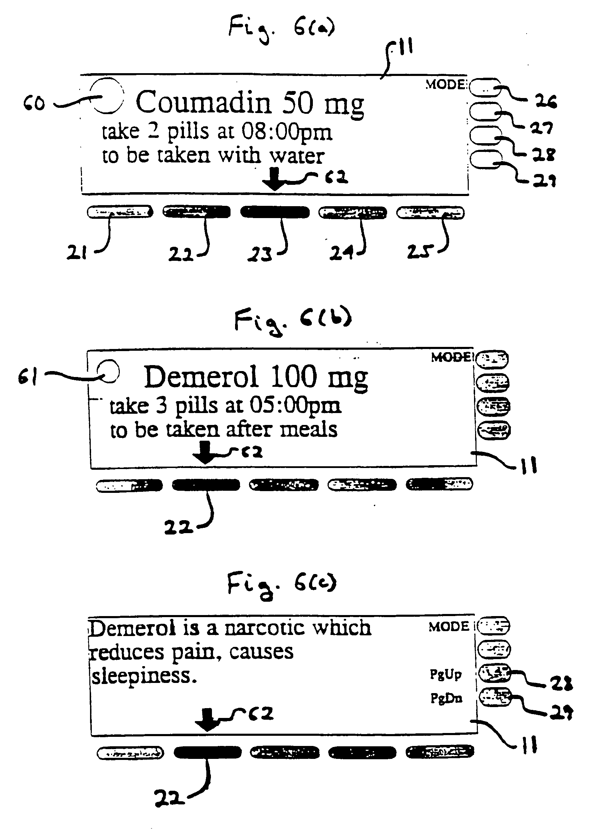

From the passive mode 61, the patient can readily obtain information concerning any of the medications contained in the medication compartments by actuating or pressing the dedicated key associated with the medication regarding which the information is sought. When the patient actuates or presses a dedicated key one time, the device displays a prescription label message concerning the medication contained in the medication compartment associated with the actuated key. For example, as illustrated in Fig. 6(a), if the patient actuates dedicated key 23, the device displays the name of the medication contained in the associated medication compartment 33 ("Coumadin 50 mg"), the prescribed schedule for that particular medication ("take 2 pills at 08:00pm"), a graphic representation and indication of the color of the medication 60, information concerning the optimum conditions for taking the medication ("to be taken with water"), and an arrow 62 pointing to the dedicated key actuated and associated medication compartment. If the patient does not actuate any other keys for a predetermined wait period (preferably approximately from 5-30 seconds), then the device returns to passive mode 61.

If the patient then actuates a different dedicated key, either within the predetermined wait period or after that period of time, the device displays the label message concerning the medication contained in the medication compartment associated with that key. For example, as illustrated in Fig. 6(b), if the patient actuates dedicated key 22, the device displays the name of the medication contained in the associated medication compartment 32 ("Demerol 100 mg"), the prescribed schedule for that particular medication ("take 3 pills at 05:00pm"), a graphic representation and indication of the color of the medication 61, information concerning the optimum conditions for taking the medication ("to be taken after meals"), and an arrow 62 pointing to the actuated dedicated key and associated medication compartment. Then, as specified above, if the patient does not actuate any other keys for a predetermined wait period, the device returns to passive mode.

If the patient actuates the same dedicated key 22, within the predetermined wait period relative to the first actuation, then the device displays additional information concerning the medication contained in the associated medication compartment. For example, as illustrated in Fig. 6(c), if the patient actuates dedicated key 22 within the predetermined wait period relative to the first actuation, the device displays descriptive information concerning the medication contained in the associated medication compartment (Demerol). Also, the controller assigns page up and page down functions to the soft function keys 28 and 29, respectively. Actuation of the page up and page down soft function keys 28 and 29 causes the microcontroller 10 to scroll through screen displays containing additional information concerning the associated medication. Then, as specified above, if the patient does not actuate any other keys for the predetermined wait period, the device returns to passive mode.

Further successive actuations of a same dedicated key will access additional information concerning the medication contained in the medication compartment associated with the actuated key. The additional information may include information about the particular medical condition that the medication treats (i.e. general information about the medical condition, information about how the medication impacts the condition, and information about the patient's prognosis based on adherence to the prescribed medication schedule). Further information may concern potential side effects and adverse reactions associated with the particular medication, the process of diagnosing or treating the medical condition, harmful or adverse interaction with other medications, and laboratory procedures or other diagnostic tests involved in diagnosing and treating the medical condition.

Also from the passive mode 61, an alarm may activate signaling the patient through visual and/or audible alert signals. The alarm may either be a scheduled medication alarm, or a general alarm. The scheduled medication alarm alerts the patient and indicates that certain scheduled medication doses are due to be taken. The general alarm alerts the patient and indicates that some other action is required. The other action may be one of several possibilities, including response to certain patient queries, performance of a particular test, placing the device within the cradle for communication with a remote device, phoning or making an appointment with the health care provider, giving an injection, inhaling an aerosolized medication, etc.

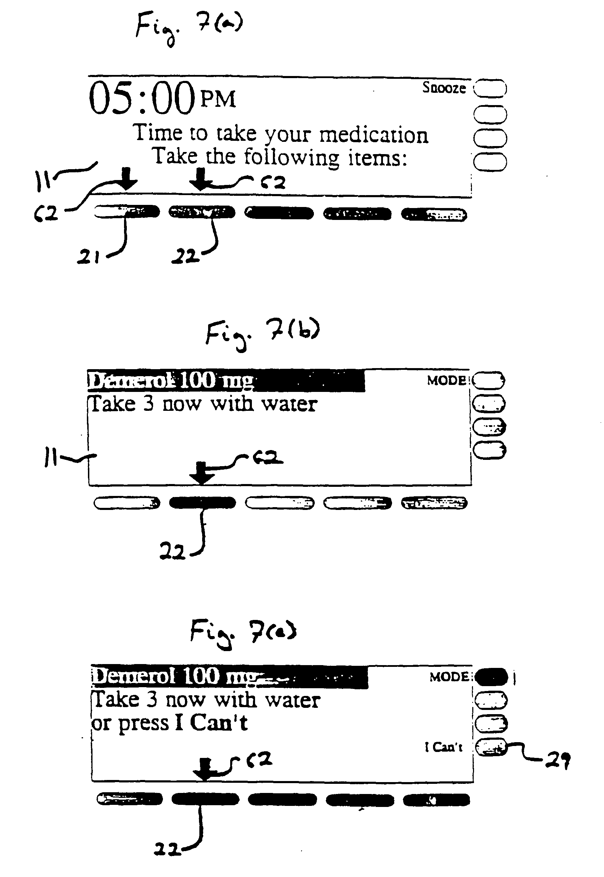

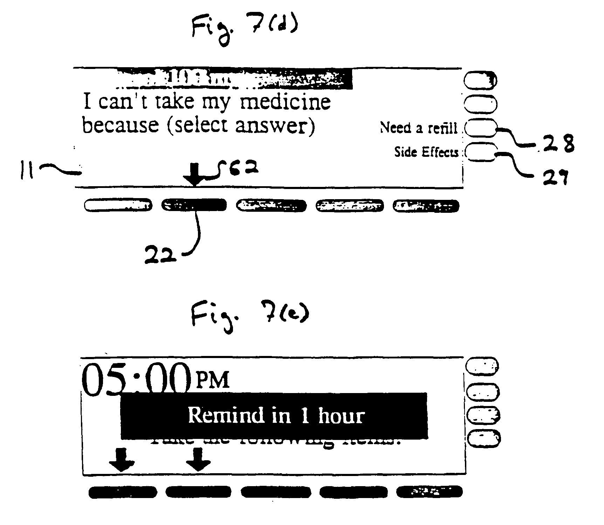

Referring to Fig. 7(a), when a scheduled medication alarm occurs, the device continues to display the current time, and displays a message indicating that scheduled medication(s) are due to be taken. The device designates the particular scheduled medications with arrows 62 pointing to the appropriate medication compartments. The patient can access specific information concerning the medications that are the subject of a scheduled medication alarm by actuating the dedicated key associated with one of those medications. Further, during a scheduled medication alarm, the microcontroller 10 assigns a snooze function to the soft function key 26, and actuation of the snooze key suspends the alarm for a predetermined period of time, previously programmed into the device (i.e. 1 hour), as illustrated in Fig. 7(e). The snooze function defaults to 15 minutes if not overridden during device programming. As illustrated in Fig. 7(b), upon actuating dedicated key 22, the device displays the name and strength of the medication in he upper left corner of the LCD 11 in reverse video, the prescription requirements for the associated medication, a request that the patient take the prescribed quantity at that time and any special instructions ("Take 3 now with water").

When the patient opens the appropriate medication compartments during a scheduled medication alarm, the compartment switch associated with each compartment signals the microcontroller 10. The microcontroller 10 then displays a message on the LCD 11, designating the prescribed quantity for the medication in the opened compartment and the conditions for taking the medication, as illustrated in Fig. 7(c). The message requests that the patient either take the designated quantity prescribed for the medication or alternatively actuate the soft function key 29 to indicate that the medication cannot be taken, as illustrated in Fig. 7(c).

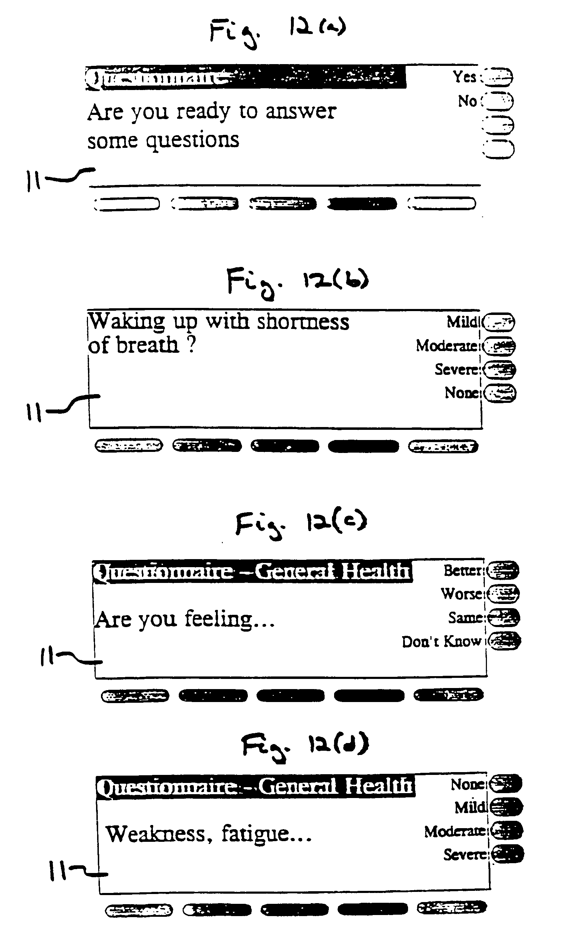

Then, actuating the "I Can't" soft function key 29, signals the microcontroller 10, which displays an appropriate message. For example, as illustrated in Fig. 7(d), the message queries the patient as to why the scheduled medication cannot be taken, with the potential responses of "Need a refill" and "Side Effects" assigned to soft function keys 28 and 29, respectively. Actuating the soft function key assigned to a particular response signals the microcontroller 10 and indicates the patient's response, and the microcontroller 10 records the response along with other associated information (i.e. the particular medication to which the response relates and the time of day when the response was given). Actuating the soft function key 29 indicating that the patient is experiencing adverse reactions or side effects may then prompt the microcontroller 10 to query the patient as to the particular reactions or effects experienced, as illustrated in Figs. 12(a)-12(d). The microcontroller 10 will again record the patient's response along with other associated data.

Opening and closing a designated compartment, during a scheduled medication alarm, signals the microcontroller 10, indicating that the patient adhered to the prescribed schedule for the medication contained in the compartment, The microcontroller 10 then records the patient's adherence accordingly. If the patient fails to obey a scheduled medication alarm and does not open and close each of the designated medication compartments, within a predetermined period of time or until the next scheduled medication alarm, then the alarm will continue at periodic intervals (i.e. 15 minutes). The alarm will also provide a visual signal (i.e. "MISSED.MEDICATION"), informing the patient that a scheduled medication was not taken, and designating the appropriate compartment and necessary quantity- The device may display other messages on the LCD 11, warning the patient of the adverse consequences associated with the failure to adhere to the particular medication schedule. The scheduled medication alarm will deactivate once the patient accesses all of the designated medication compartments associated with the particular alarm.

The present invention provides a method that automatically adjusts the patients treatment regimen comprising the patient's scheduled medication times and doses based on patient compliance data, or other monitored aspects of the patients's treatment progress. With patients taking medication for chronic and/or acute illnesses, where a healthcare provider prescribes medication at fixed intervals or specific times, while it is preferable that patients take the medications at the prescribed intervals, patients may fail to adhere to the prescribed schedules- At other times, it is more convergent for patients to take a dose earlier than prescribed. For example, at a prescribed dosing time, the patient may be unable to take a dose because of a scheduling conflict, or for some other reason, the patient may need to take the medication earlier than prescribed.

In certain instances, is critical, however, for patients to take all of the prescribed doses for a given time period. Taking all of the doses of certain drugs in a given time period optimizes the efficacy of drug therapy in treating the underlying illness or illnesses. Further, There should not be too short or too long of an interval between doses. Too short an interval can cause unacceptable, even lethal side effects or adverse drug reactions, and too long an interval between doses can cause unacceptable break-through symptoms, a worsening of the illness, re-hospitalization, or even death.

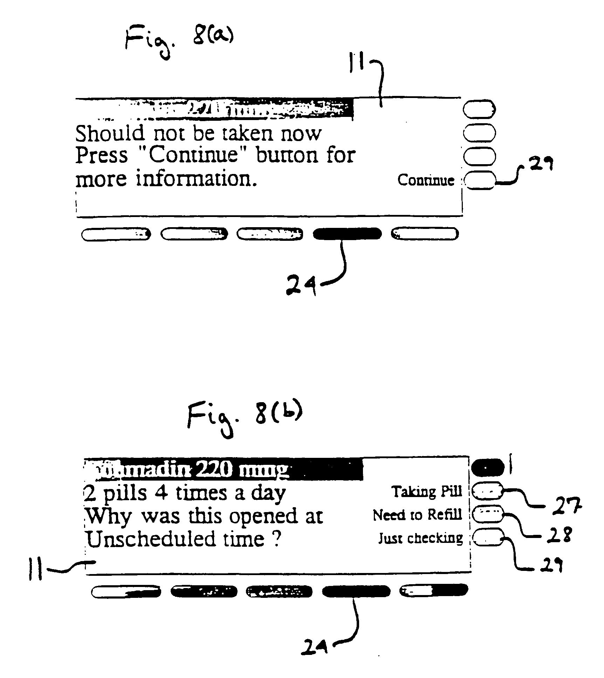

An algorithm or algorithms are provided that automatically adjust subsequent dosing intervals when the medical monitoring device determines a need for such adjustments to safely, properly, and optimally administer the remaining doses. For example, the medical monitoring device of the present embodiment would include an algorithm for calculating the next scheduled dose time based on the timing of a current dose. If the patient opens a compartment outside of a specified window of time for a scheduled dose, or while the device is in the passive mode as described above, the device provides audible and visual signals indicating that the patient is accessing an unscheduled dose, referring to Figs. 8(a) and 8(b). If the patient then actuates the "continue" soft function key 29, and then the "Taking Pill" soft function key 27, the device would record information concerning the unscheduled dose. Additionally, the device would execute an algorithm that analyzes the timing of the unscheduled dose with regard to the previous and next scheduled doses for that medication, and calculate a modified next scheduled dose accordingly. The medical monitoring of the present embodiment could further include algorithms that analyze monitored data, as described in detail above, and adjust subsequent dosing schedules based on actual patient progress and/or reactions to previous doses, or based on any other monitored aspects of the patient's physical health and symptoms. The device of the present embodiment will optimize the patient's dosing intervals; thereby, reducing side effects, adverse reactions, and illness symptom break-through; resulting in optimized medical treatment and quality of life.

A medical monitoring device provides access to refrigerated or frozen medications. In at least one of the medication compartments, the device stores tokens or keys that provide access to a refrigerated container storing prescribed medication(s) to be taken- when a scheduled medication alarm occurs, the device indicates that a token or key should be removed from a particular compartment, and that token or key in turn provides access to the appropriate refrigerated container storing the medication to be taken. Alternatively, the device electronically provides access to the refrigerated container- In such a case, when a scheduled medication alarm occurs, the device indicates a particular compartment to open. Upon opening that compartment, the device transmits, through a wireless means, a signal that unlocks the appropriate refrigerated container providing access to a prescribed medication that is the subject of the alarm. The wireless means can comprise any electronic transmission method known to those skilled in the art, i.e., infrared or a radio frequency transmission.

The audible scheduled medication alarm signal includes the use of a transducer or voice generation chip and speaker that emits speech for giving audible instructions concerning the prescribed medications. In this embodiment, a number of common messages and/or words generally associated with prescription instructions are stored in the SRAM 13 or EPROM 12 (i.e. "Take", "Pill", "A", "Pills", "Water", "Juice", "Times", "One", "Two", "Day", etc.). The messages and/or words are stored in a table look--up format, and the device plays audible messages by sequencing through specific combinations (i.e. "Take Two Pills With Milk Two Times A Day"). Also, additional messages specific to particular medications could be programmed into the SRAM 13 or EPROM 12 for alerting the patient under certain predefined circumstances.

When the patient opens a medication compartment while the device is in the passive mode and no scheduled medication alarm is occurring, an unscheduled pill alarm occurs. During an unscheduled pill alarm, the device provides audible and visual signals. Referring to Fig. 8(a), the visual signals comprise a displayed message, with the medication label and strength in reverse video at the upper left corner of the LCD 11, along with a message that the medication should not be taken at that time. The device also displays a message indicating that the patient should actuate the "continues soft function key 29 for more information. Actuating the "Continue" soft function key initiates the display of the medication label and strength in reverse video at the upper left corner of the LCD 11, along with the prescribed medication schedule for the medication contained in the opened compartment. The device also displays a query as to why the compartment was opened, with potential responses assigned to particular soft function keys.

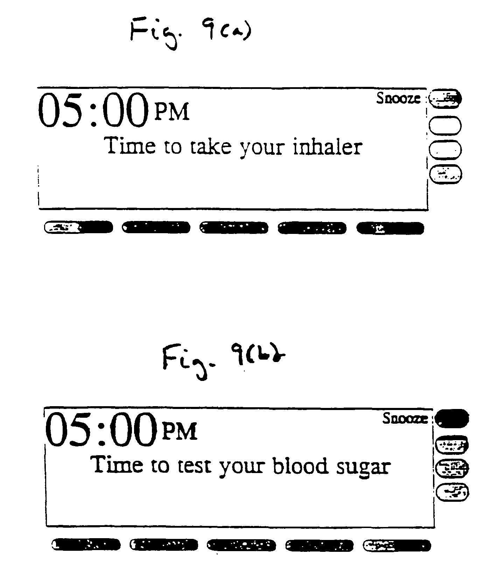

Other alarms occurring from the passive mode alert the patient concerning other medical related activities. For example, as illustrated in Fig. 9(a), an alarm can alert the patient that a dose of an inhaler is due to be taken. As illustrated in Fig. 9(b), additional alarms can remind the patient to perform certain tests, such as a blood sugar test.

A selectable sound and/or light alarm means is provided that presents audible and/or visible signals indicating messages or instructions to the patient- For example, during a medication alarm, the medical monitoring device provides audible and/or visible signals. The audible signals may comprise music, a melody or a series of tones whereby, if the patient opens a wrong compartment during a medication alarm, then the device would play a sad or disturbing piece of music, melody or series of tones. If the patient opens the proper compartment(s) during a medication alarm, then the device would play a happy or pleasing piece of music, melody or series of tones. Further, the visible signals may comprise lights, whereby, if the patient opens a wrong compartment during a medication alarm, then the device would illuminate or display a red light indicating "stop", or a yellow light indicating "caution." If the patient opens the proper compartment(s) during a medication alarm, then the device would illuminate or display a green light indicating "go." The audible and/or visible signals would be selected during the programming of the device by a healthcare provider, or by a user during execution of the programming mode of the medical monitoring device. The medical monitoring device of the present invention thereby provides a means whereby a patient suffering from an incapacitating condition (i.e., senility, Alzheimer's, organic brain disease, etc.) could operate the medical monitoring device and understand messages or instructions conveyed by the device. Further, many other possible means may be employed for communicating with an impaired patient, including vibration.

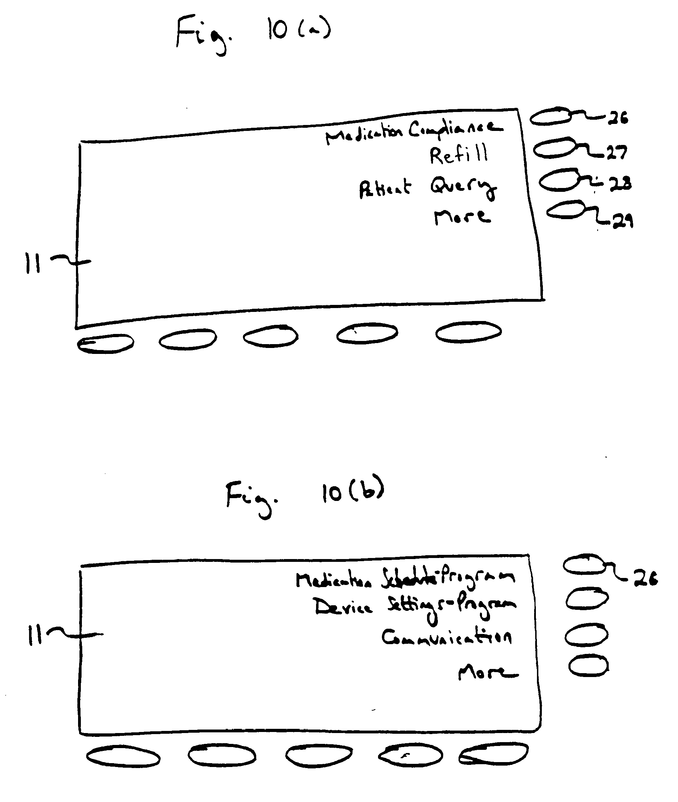

Further, while in the passive mode 61, soft function key 26 controls a mode-select function that initiates an active mode of operation whereby the patient initiates different operating modes of the device. Referring to Fig. 10(a), actuating the mode-select soft function key 26 from the passive mode displays a mode-select screen, wherein each soft function key is assigned a different mode-select function. Soft function key 26 initiates a "Medication Compliance" mode, soft function key 27 initiates a "Pour" mode (for refilling medication compartments), soft function key 28 initiates a "Patient Query" mode, and soft function key 29 is assigned a "More" function. Actuation of the "More" soft function key 29 scrolls the display for accessing additional modes of operation, as illustrated in Fig. 10(b). The additional modes of Fig. 10(b) are a "Medication Schedule - Program" mode, a "Device Settings - Program" mode, and a "Communication" mode.

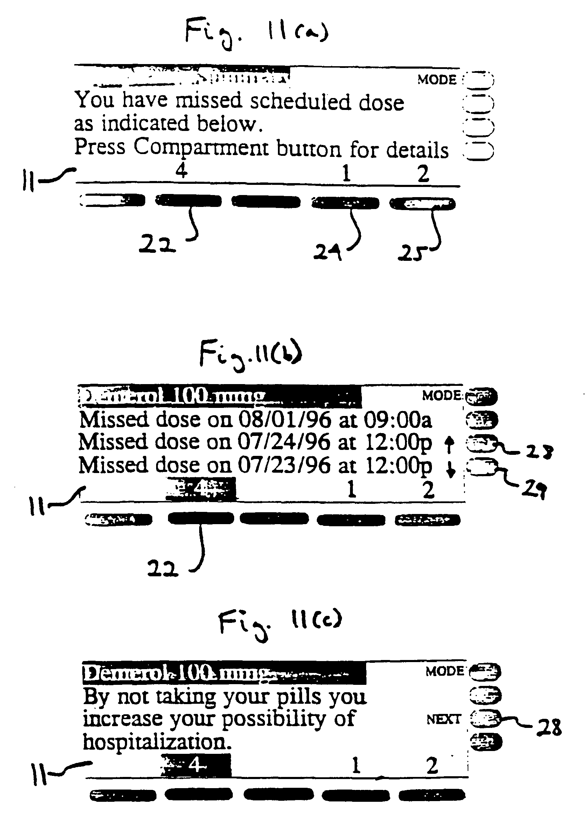

Referring to Fig. 11(a), in the "Medication Compliance" mode, the device displays a summary screen with the title "Compliance Summary" displayed in reverse video in the upper left corner, and a synopsis of the patient's medication schedule adherence. Specifically, above each medication compartment containing a medication for which the patient missed scheduled doses, the device displays the number of missed doses for the medication contained in the associated compartment. For example, as illustrated in Fig. 11(a), the patient missed 4 doses of the medication in compartment 32 (the compartment associated with dedicated key 22), 1 dose of the medication in compartment 34 (the compartment associated with dedicated key 24) and 2 doses of the medication in compartment 35 (the compartment associated with dedicated key 25) .

The patient then actuates a particular dedicated key to obtain the details concerning the missed schedules for the medication contained in the associated compartment. For example, as illustrated in Fig. 11(b), when dedicated key 22 is actuated, the device displays the name and strength of the associated medication in reverse video in the upper left corner of LCD 11, and displays messages indicating that the patient missed certain doses at specified dates and times. In this instance, since only three missed doses fit on the LCD screen, soft function keys 28 and 29 serve as scroll keys for scrolling up and down to review additional entries of missed medication doses. Further actuation of the dedicated key provides the patient information concerning the potential or likely consequences of missing the medication dose, as illustrated in Fig. 11(c). Additionally, soft function key 28 serves a "NEXT" function for accessing additional information concerning the missed doses of the particular medication, and for accessing information concerning unscheduled medication doses taken by the patient. The device thereby provides educational information for motivating the patient to comply with the medication schedules.

Referring to Figs. 12(a)-12(d), in the "Patient Query" mode, the device displays a series of questions concerning health status assessment, adverse effects or side effects experienced by the patient, treatment progress and the patient's general quality of life. Other questions that require responses from the health care provider may be posed. The questions are framed in either true/false (yes/no) format, scaling format or multiple choice format, and the soft function keys 26-29 are assigned particular responses according to the question format. Actuating a soft function key signals the microcontroller 10 and indicates the particular response chosen, and the microcontroller 10 records the patient's or health care provider's responses along with associated data (i.e. date and time of questioning). Questions may also be framed in decision tree format depending on the extent and complexity of the details necessary for the most efficient administration of medication, or administration of other components of the medical treatment plan, for treating a particular medical condition.

The "Communication" mode facilitates the transfer of data between the medical monitoring device and a remote device. During the communication mode the health care provider can download specific programming data associated with a particular patient's prescribed medication schedule and medical treatment regimens, and upload patient response data or other information, through the communications port 17.

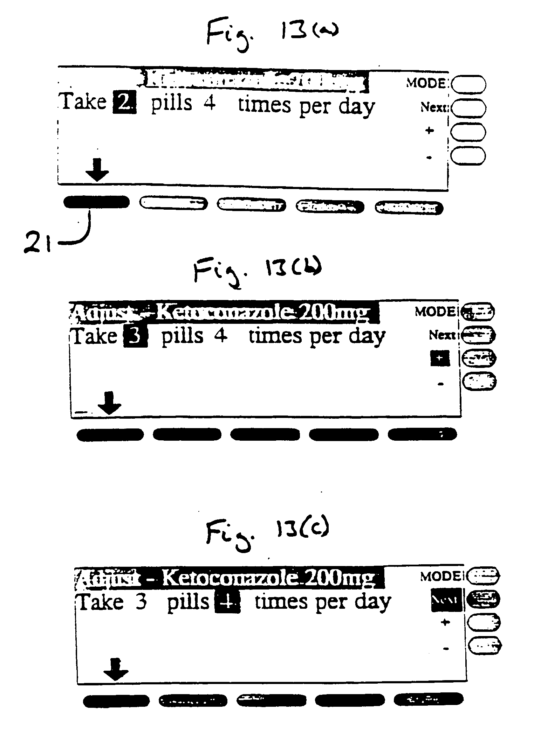

Further, referring to Figs. 13(a)-13(f), in the "Medication Schedule - Program" mode, the device allows the patient or health care provider to modify prescribed medication schedules for a particular medication. Actuating the dedicated key 21, while in the "Medication Schedule - Program" mode, facilitates the modification of the prescribed schedule for the medication contained in the associated medication compartment. The device displays an adjust message, with the name and strength of the medication being adjusted, in reverse video in the upper left corner of the LCD 11, and displays the current prescribed frequency ("Take 2 pills 4 times per day"), as illustrated in Fig. 13(a). The number of pills per dose "2" is highlighted, and actuating the "+" soft function key 28 or the "-" soft function key 29 raises or lowers the highlighted number by one for each actuation, accordingly. Fig. 13(b) illustrates the display after actuating the "+" soft function key 28 one time; the number of pills per dose is now "3" instead of "2" ("Take 3 pills 4 times per day"). Actuating the "Next" soft function key highlights the next setting (the frequency or doses per day) for modification, as illustrated in Fig. 13 (c), and that setting is modified in the same manner.

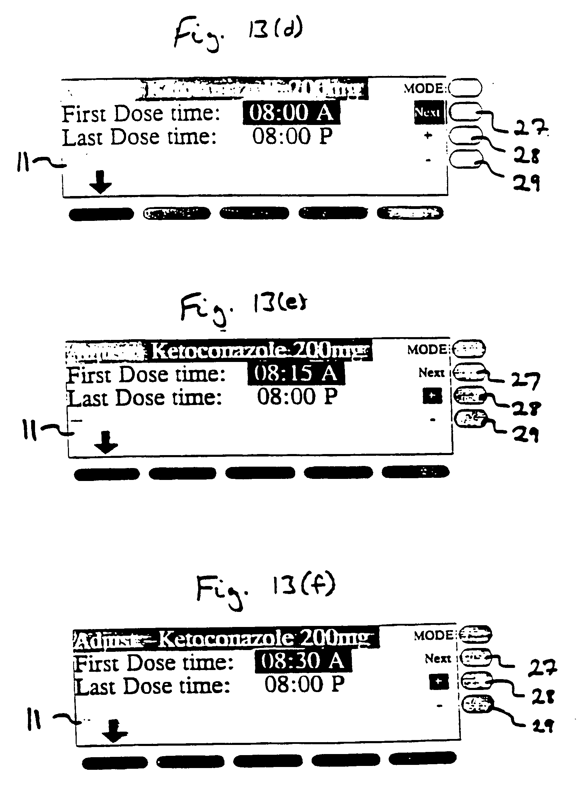

The user can then set the dose times, by actuating the "Next" soft function key 27, as illustrated in Figs. 13(d)-13(f). Referring to Fig. 13(d), the first dose time and last dose time are displayed on the LCD 11, with the first dose time highlighted. The first dose time is changed in 15 minute increments by actuating the "+" and "-" soft function keys 28 and 29. The device automatically calculates the last dose time by adding 12 hours to the first dose time, and sets the intermediate dose times by dividing the 12 hour period by the scheduled number of doses per day. As illustrated in Fig. 13(e), actuating the "+" soft function key 28 one time increases the first dose time 15 minutes to "08:15 A", and, as illustrated in Fig. 13(f), actuating the "+" soft function key 28 another time increases the first dose time another 15 minutes to "08:30 A".

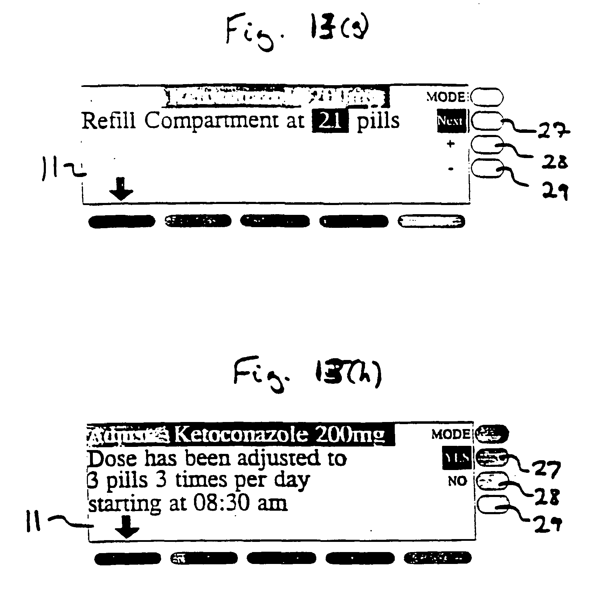

After setting the dose times, actuating the "Next" soft function key 27 allows for the modification of the refill amount, as illustrated in Fig. 13(g). The refill amount is highlighted on the LCD 11, and the "+" and "-" soft function keys 28 and 29 increase and decrease the refill amount, accordingly.

Referring to Fig. 13(h), after completion of the "Medication Schedule - Program" mode, the device displays the new settings, and requests that the user verify them by actuating the "Yes" soft function key 27. If the settings are incorrect, the user actuates the "No" soft function key 28, and the device returns to the beginning of the "Medication Schedule - Program" mode.

Further, the mode soft function key 26 enables the user to modify certain device settings or program information (i.e. date and time of day settings, alarm volume, snooze interval, etc.).

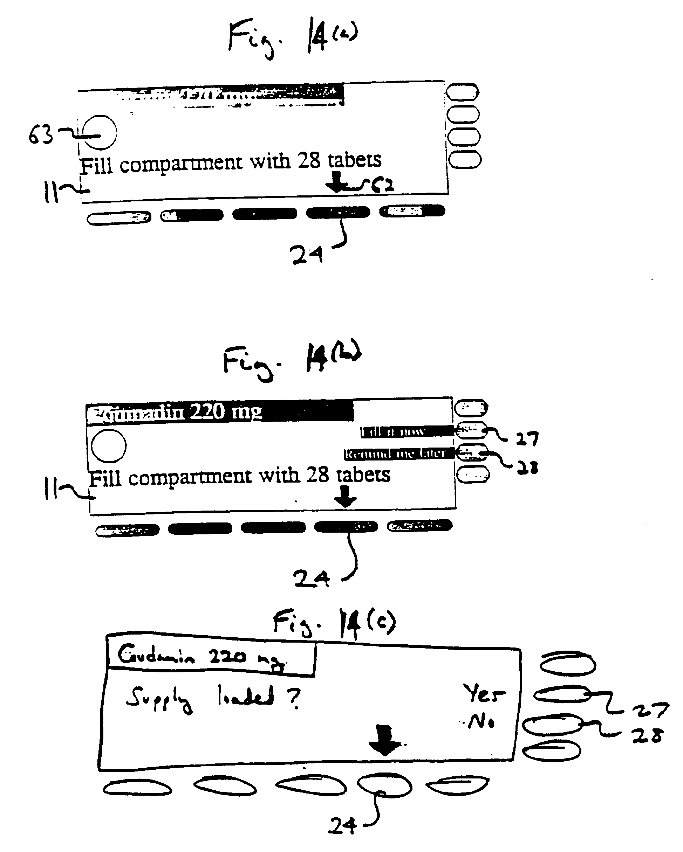

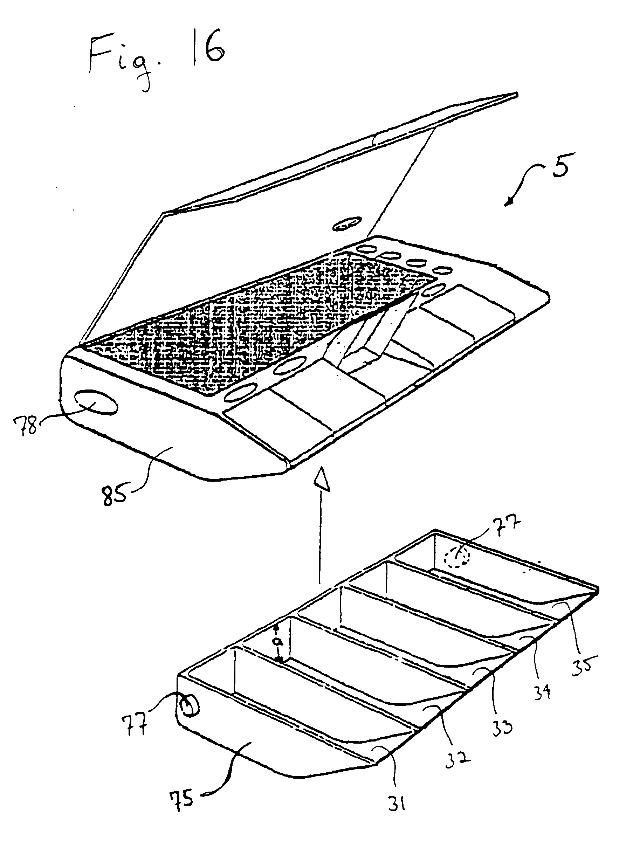

The device tracks the number of pills in each compartment as the patient complies with scheduled medication alarms. From the passive mode, when the device figures that a medication compartment is empty, the device provides a "Refill" alarm to alert the patient that it is time to refill a medication compartment, as illustrated in Figs. 14(a)-14(c). Referring to Fig. 16(a), upon the occurrence of a "Refill" alarm, the device displays the name and strength of the medication to be refilled in reverse video in the upper left corner of the LCD 11, along with a graphic representation of the medication 63. The device also indicates the number of pills to be refilled and displays an arrow pointing to the appropriate medication compartment (the medication compartment 34 associated with dedicated key 24).

The alarm continues until the user actuates the associated dedicated key 24. The device then allows the user to actuate the "Fill it now" soft function key 27, or the "Remind me later" soft function key 28, as illustrated in Fig. 14(b). If the user actuates the "Remind me later" soft function key 28, then the "Refill" alarm will occur at a preprogrammed future amount of time (the default is 2 hours). The "Refill" alarm will continue periodically until the user actuates the "Fill it now" soft function key 27, or unless the associated medication compartment is programmed to be empty. Actuating the "Fill it now" soft function key 27 redisplays the previous screen, and the associated medication compartment must be opened and closed to deactivate the "Refill" alarm. Once the associated medication compartment is opened and closed, the device prompts the user to verify the supply load, as illustrated in Fig. 14(c). If the user verifies the supply load by indicating "Yes", then the device will continue tracking the inventory of the associated medication compartment. Otherwise, if the user verifies the supply load by indicating "No", then the device disables the inventory tracking function for the associated medication compartment.

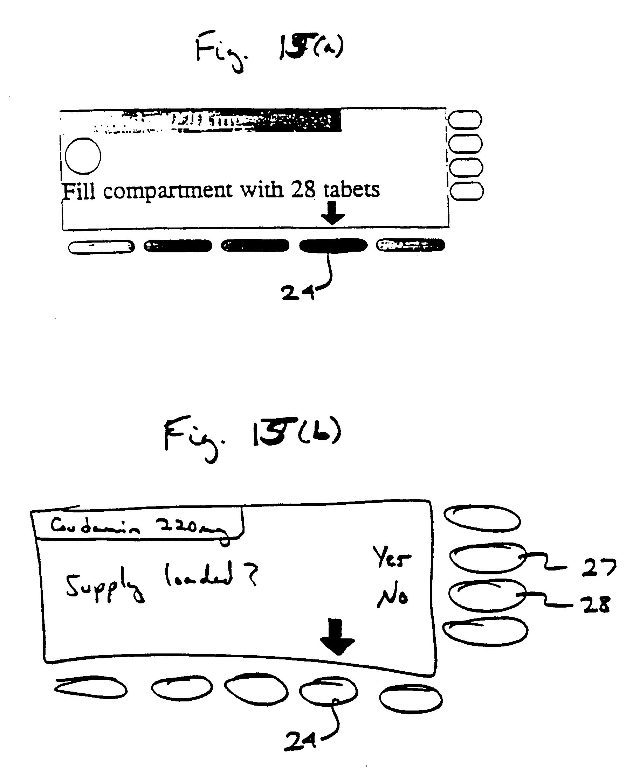

Referring to Fig. 10(a), the "Refill" mode can be selected by actuating the "Refill" mode soft function key 27, when the device is in the active mode (after actuation of the mode-select soft function key 26, from the passive mode) . After user initiation of the refill" mode, the user must actuate the dedicated key associated with the medication compartment to be refilled, and may be prompted to do so by a message on the display. As illustrated in Fig. 15(a), the device then displays the name and strength of the medication to be refilled in reverse video in the upper left corner of the LCD 11, along with a graphic representation of the medication 63. The device also indicates the number of pills to be refilled and displays an arrow pointing to the appropriate medication compartment (the medication compartment 34 associated with dedicated key 24). Once the associated medication compartment is opened and closed, the device prompts the user to verify the supply load, as illustrated in Fig. 15(b). If the user verifies the supply load by indicating "Yes", then the device will continue tracking the inventory of the associated medication compartment. Otherwise, if the user verifies the supply load by indicating "No", then the device disables the inventory tracking function for the associated medication compartment.

The medical monitoring device 5 provides for the use of a variable capacity compartment tray and storage system that affords complete flexibility in the quantities and volumes of solid, liquid or aerosolized doses contained within the unit. The variable capacity compartment tray also provides for pre-loading, and pre-labeling of medications. Accordingly, when the patient exhausts a supply of medication in one tray, a second pre-loaded and pre-labeled tray can quickly and easily replace the exhausted tray.

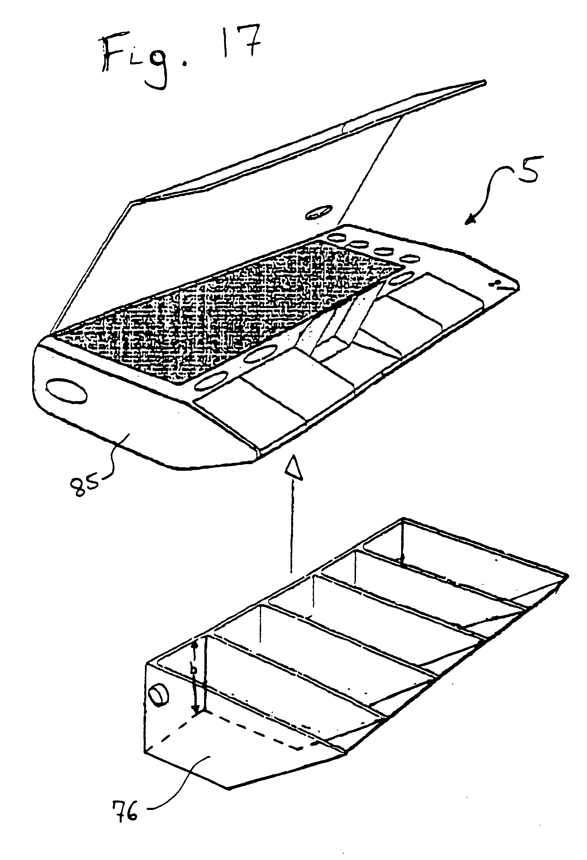

As illustrated in Fig. 16, the medical monitoring device 5 includes a removable tray 75 that can easily be inserted and removed. The tray 75 contains the medication compartments 31-35. The tray 75 has extensions 77 protruding from each side that insert into corresponding slots 78 in the top portion 85 of the device 5. The tray 75 is a predetermined depth (a). A tray 76, as shown in Fig. 17, can be of a different predetermined depth (b). The differing depths provide for variable quantities, volumes and types of medications necessary for a given treatment regimen.

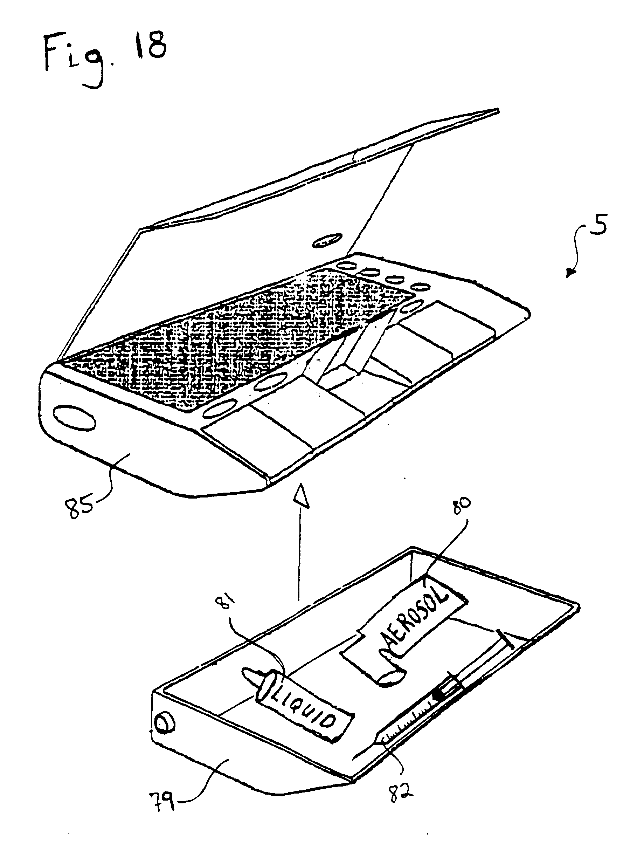

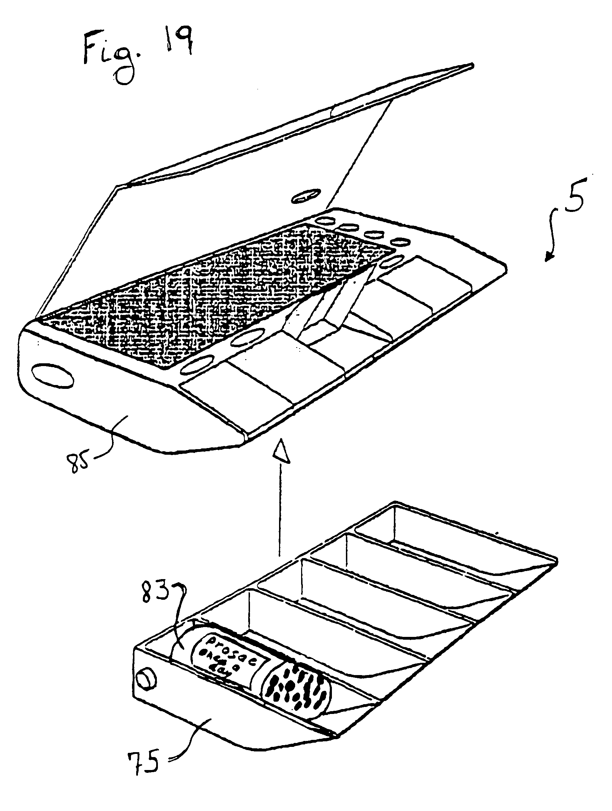

The predetermined depth is based on functionality. The compartments of a particular tray must be capable of storing doses of different types of medication covering a certain time period. For instance, each compartment of one size tray may be designed to store 84 tablets of a medication provided in a tablet size of 1 centimeter in diameter by 0.5 centimeters thick. The tray could store a week's supply of such a medication, where three pills are prescribed to be taken four times a day. In addition to the one medication, another compartment of the same tray could store a larger supply of another medication provided in a smaller tablet size. Various size trays can be provided, each capable of storing a number of different combinations of different medications subject to different prescription schedules and covering various time periods. Further, a tray 79 can contain compartments capable of storing a canister 80 for a medication in the form of an inhalant, a container 81 for a medication in the form of a liquid, or pre-loaded syringes 82 for medication taken by injection, as illustrated in Fig. 18. Additionally, as illustrated in Fig. 19, the tray 75 can be of a depth that accommodates a medication bottle 83 as dispensed by a pharmacist. Additionally, trays or compartments can provide direct storage of liquid, injectable, or aerosolized medications.

The trays of varying sizes are provided to accommodate various volumes of multiple solid dose medications. For example, at one time the user might wish to have a compact design for maintaining a one to three day supply of medication. At another time the user might be traveling for an extended period of time, and thus would require a larger volume tray for accommodating a week's supply or longer of various medications. The user could then replace the compact tray with a larger volume tray containing a larger supply of medication. The device of the present embodiment thereby provides flexibility to meet the particular requirements of the user at any given time.

Each tray includes a mechanism for attaching to the top portion 85 of the device 5, which houses the electronics and user interface. The attaching mechanism can be any mechanical device suitable for easy insertion of the trays into, and removal of the trays from, the medical monitoring device. Such mechanisms are within the purview of ones reasonably skilled in the art

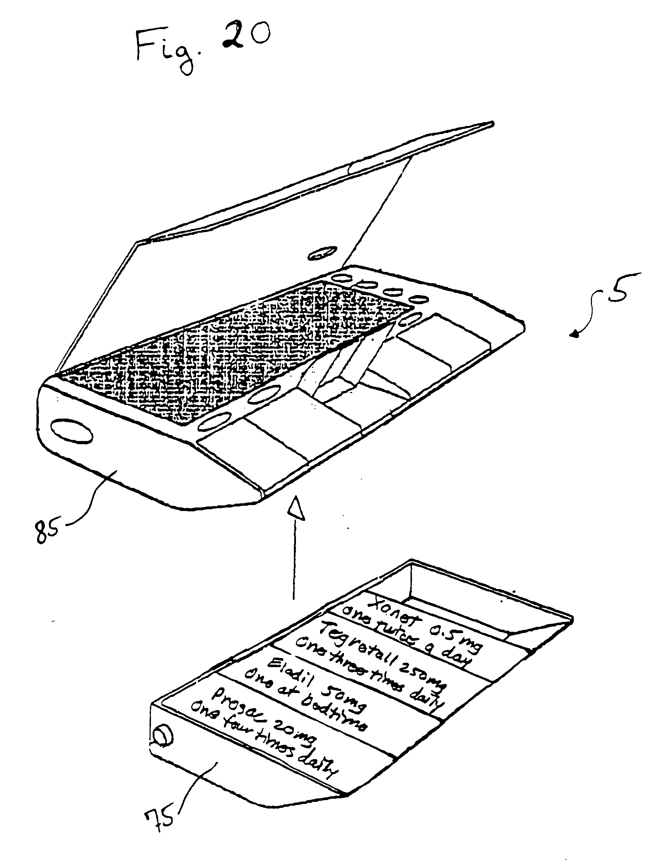

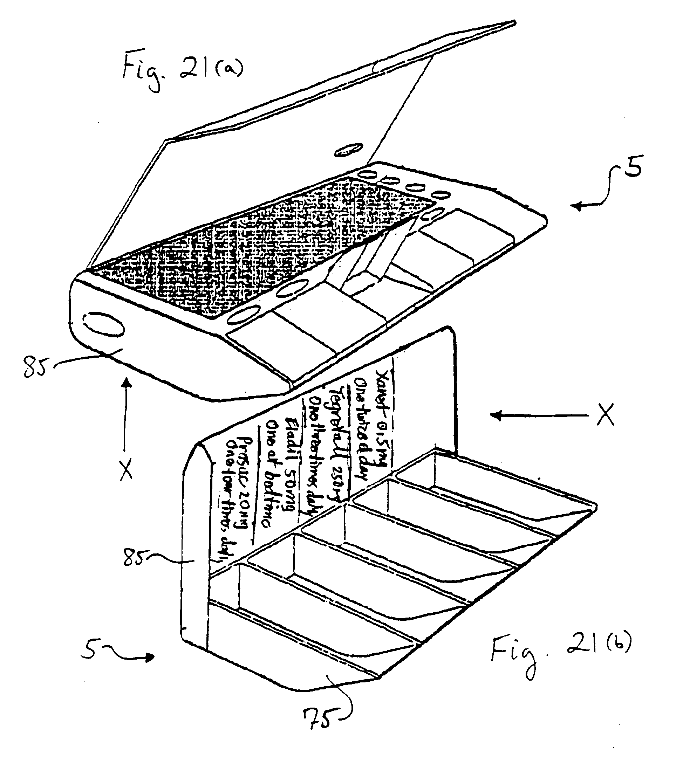

Each tray can be independently labeled by the pharmacist, with labels being placed on a top lid for each compartment within the tray, as shown in Fig. 20. Additionally, or alternatively, labels could be placed on the underside of the top portion 85 of the device, as shown in Fig. 21 (b), which is a view in the direction X of the device 5 as depicted in Fig. 21 (a) .

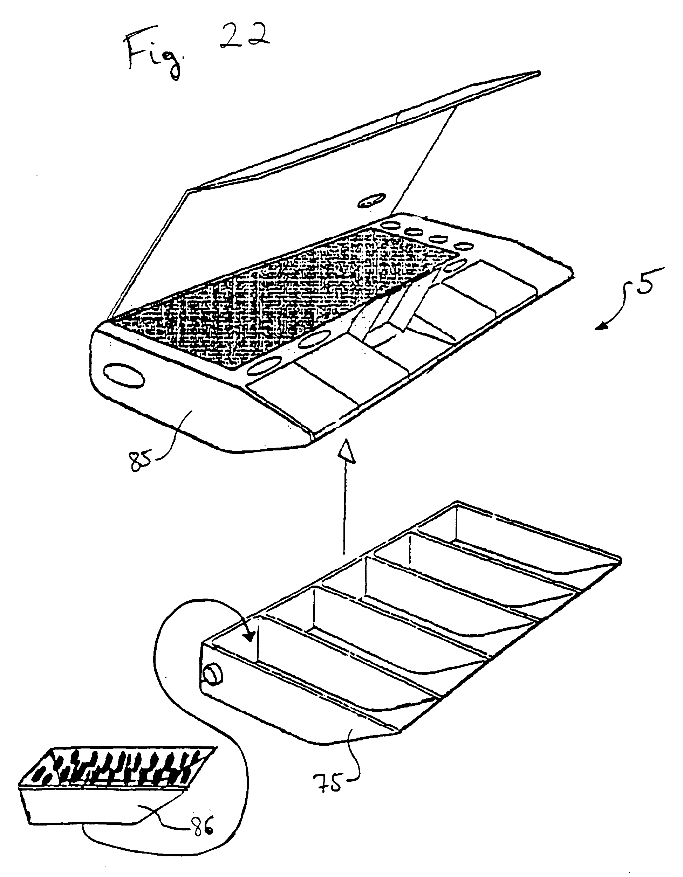

As illustrated in Fig. 22, the tray 75 accommodates a series of liners 86 that fit into each compartment. Each liner holds a prepackaged unit dosage of medications. This embodiment enables the pharmacist to fill, seal and label the liners, so that the user then need only insert a full liner in its respective compartment in the tray 75. Additionally, the label can be reused by peeling it off the liner 86 and placing it on the lid for the respective compartment (as shown in Figure 20) or placing it on the underside of the top portion 85 of the device (as in Fig. 21 (b)).

The tray and labeling mechanism provides for increased accuracy and efficiency, and reduces errors occurring when patients reload the medical monitoring device.

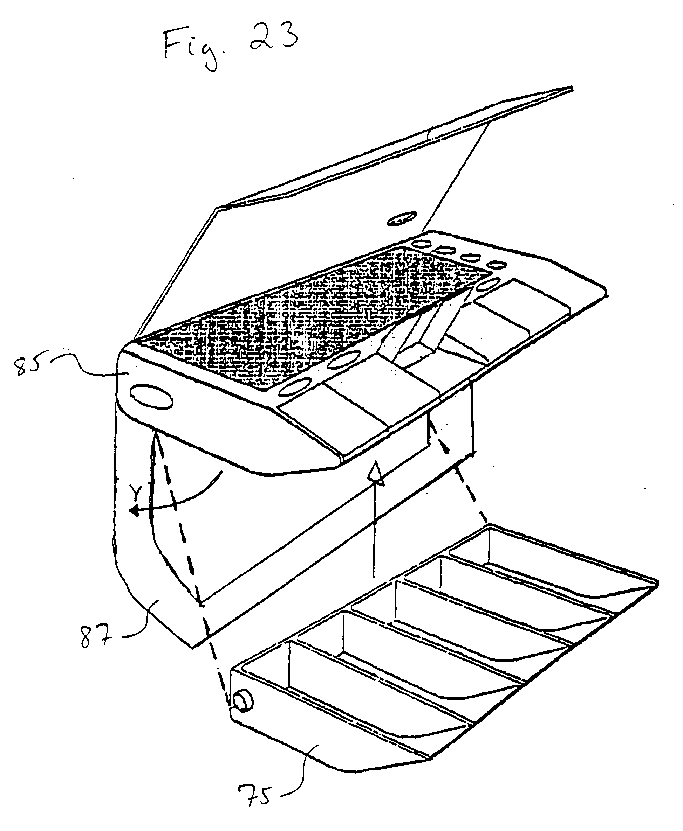

As illustrated in Fig. 23, the top portion 85 of the device includes a frame or support cover 87 that is connected by a hinge mechanism- The frame or support cover 87 unlatches from the top portion 85 and rotates in the direction of the arrow Y, allowing for insertion of the tray 75. The frame or support cover 87 then rotates back and latches into the top portion 85, holding the tray 75 within the device.

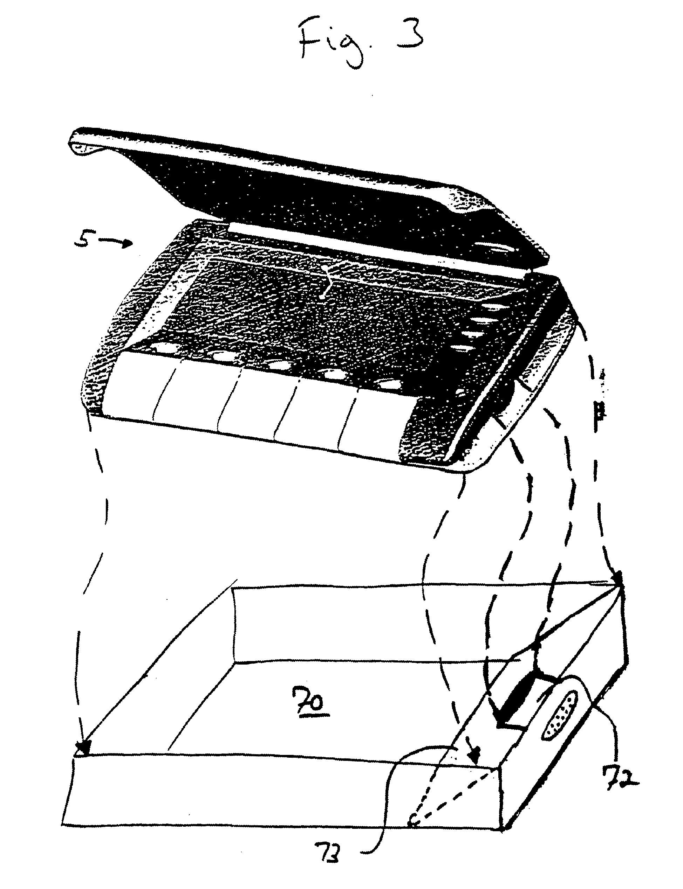

As illustrated in Fig. 3, an electronic cradle 70 is provided for amounting the device 5 to facilitate automatic communications, programming and uploading. The cradle includes an interface 72 capable of transmitting data to, and receiving data from, the communications port 17. The cradle further includes a modem device 73. The cradle's modem device 73 can be programmed to automatically dial certain numbers at certain times and connect to remote devices, or be instructed to do so by the medical monitoring device. Once connected to a remote device, the cradle then provides patient identification information to the remote device, and either downloads programming data from the remote device, or uploads user response data to the remote device, or performs both downloading and uploading, automatically. The present invention thereby provides health care providers with the ability to automatically download a program to the patient's device, and upload information concerning the patient's treatment progress, including data reflecting compliance with prescribed treatment, from a remote location.

A method and means are provided for wireless communication with and polling of the medical monitoring device. Referring to Fig. 1, the medical monitoring device includes a receiver-transmitter 8 connected to the microcontroller 10, and an antenna 7 connected to the receiver-transmitter 8 for receipt and transmission of wireless communication signals. The wireless communication may be by a cellular telephone link, satellite link, or other radio communication means, commonly known to those of ordinary skill in the art of electronic communications.

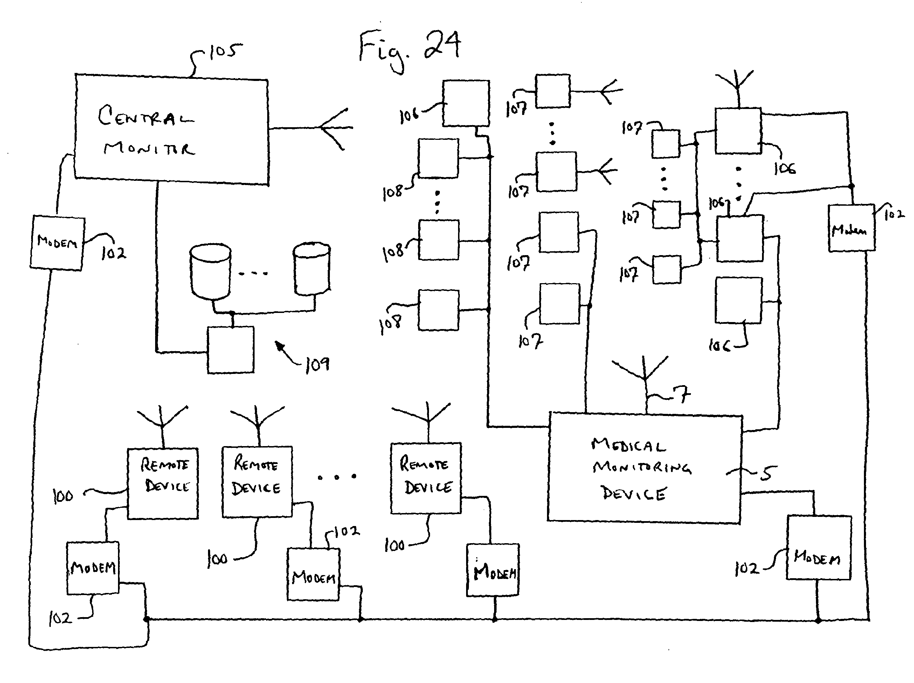

The wireless method and means of the present embodiment provides for the transmission of treatment data from a remote device 100 to the medical monitoring device, real-time monitoring of the progress of a treatment regimen being administered, and real-time monitoring of elements of a patient's health and physical status, as illustrated in Figs. 1 and 24. The real-time monitoring facilitates various tasks including, conducting a health status assessment, monitoring adverse effects or side effects experienced by the patient, monitoring treatment progress, monitoring physiologic data from serum or urine, monitoring events at the cellular, molecular and endocrine levels, and assessing the patient's general quality of life. The device is capable of receiving treatment information continuously, periodically, and upon demand by a health care provider.

Once the treatment information is received by the medical monitoring device, the device executes an interaction with the patient. The interaction may comprise any one or combination of the following: (1) educational information concerning the patient's health status, treatment progress, or other information relating to the disease(s) being treated and the treatment regimen being administered; (2) instructions to the patient (i.e., instructing the patient to modify medication dosage or schedule, instructing the patient to schedule a visit, or instructing the patient to go to the emergency room); or (3) patient, monitoring, and query data. The treatment information may further comprise any other data relating to any aspect of the patient's health or general quality of life.

In the case where the treatment information comprises patient query data, once the patient query data is transmitted to the medical monitoring device, the device either promptly executes the Patient Query mode, or stores the patient query data for subsequent execution of the Patient Query mode, depending on the status of an execution command. The execution command is either transmitted with the patient query data or stored in the medical monitoring device. The execution command is set or established by a healthcare provider, or by the device itself through artificial intelligence means, and depends upon the urgency of receiving responses to the particular queries transmitted to the device. The timing associated with the execution command is based on the disease being treated and the treatment regimen being administered. For example, if patient query data relates to a congestive heart failure condition, or if the device receives data from sensors monitoring blood pressure, EKG, cellular, molecular, and/or endocrine events related to heart function; with examples including heart rate, blood pressure, sugar level, pO₂, pH, ketones, anemia, blood, serum, urine, Ca⁺⁺, K⁺, Na⁺, etc; then the healthcare provider might set the execution command for immediate transmission of the response data. On the other hand, the patient query data may have been previously stored in the medical monitoring device (i.e., during initial programming of the device for a particular treatment regimen). After the medical monitoring device executes the Patient Query mode, the resulting response data is either stored in the medical monitoring device for subsequent transmission, or promptly transmitted to the remote device 100 for storage and analysis. The timing of the transmission of the response data depends on an execution command, as described above.

The medical monitoring device analyzes the response data by comparing it to target data transmitted with the query data, or target data previously stored in the medical monitoring device. Then, if the device determines that the response data meets certain predefined criteria, the device promptly transmits the response data to the remote device 100 for storage and analysis by the healthcare provider. The predefined criteria are set or established by a healthcare provider, and depend upon the treatment being administered and the particular response data being analyzed.

For example, if the disease being treated is a congestive heart failure condition, and a single response data point indicates that the patient is suffering moderate or severe chest pain, then the device will immediately transmit the response data to the healthcare provider. As another example, if a combination of response data points indicate that the patient is suffering from moderate shortness of breath in combination with mild leg swelling, then the device will immediately transmit the response data to the healthcare provider. As a further example, if a sequence of response data points indicates that the patient has been suffering from moderate shortness of breath, and subsequently experiences a rise in blood pressure to a systolic level of over 160 mm mercury, then the device will immediately transmit the response data to the healthcare provider based upon the sequence of events experienced by the patient and entered into the device. The same example applies to data points indicating heart rate, blood pressure, sugar level, pO₂, pH, ketones, anemia, blood, serum, urine, Ca⁺⁺, K⁺, Na⁺, etc., as specified above.

The criteria may comprise target data from which the response data should not deviate, a template with which the response data is compared, or any other method for analyzing the response data in view of some predefined base-line or range of values. Further, the response data that triggers a prompt or immediate transmission may comprise a single data element, a particular sequence of data elements, or a particular combination of data elements, as described in the examples above. The timing of the transmission depends on the disease being treated and the treatment regimen being administered, and is set or established by the healthcare provider accordingly, also as described above. Therefore, a particular health outcome, or health outcomes, will determine the timing and informational content of the transmissions. In this fashion, the outcome of medical treatment can be monitored and analyzed, compared to normative, targeted, or desired goals of treatment, and deviations from the norms or targets can trigger a modification of the monitoring of the patient as well as the treatment regimen itself.

Additionally, when the data is transmitted to the remote device 100 for analysis, a healthcare provider may not be available to respond to the data. The response data, however, potentially represents an aspect of the patient's condition that would mandate critical and prompt or immediate review, so that prompt action could be taken to examine the patient, further query the patient, change the patient's treatment regimen, or instruct the patient to go to the emergency room. In such a situation, the remote device 100 executes an algorithm that analyzes the response data alone or in combination with previously transmitted data, and promptly transmit a message to a device carried by the healthcare provider or to the healthcare provider's answering service. Again, the algorithm is developed by a healthcare provider and depends upon the particular disease(s) being treated and treatment regimen being administered. The patient and healthcare provider are thereby linked, via a wireless means, through a series of two or more devices, each of which contains algorithms that analyze information and determine the appropriate timing and content of information communicated to the healthcare provider concerning the patient's health status.

The remote device 100 is also capable of performing a polling feature whereby it polls the medical monitoring device and retrieves desired information. The desired information retrieved by the remote device may include: (1) response data; (2) physiologic data; (3) data related to cellular, molecular, and endocrine or metabolic mechanisms, as specified above; (4) data concerning the patient's adherence to a medical treatment regimen; and/or (5) any other data relating to any aspect of the patient's health or general quality of life. The remote device 100 polls the medical monitoring device by communicating via a wireless means, and retrieves the desired information from the medical monitoring device's memory. In cases where a patient requires specialized attention or treatment, the polling feature provides for continuous, periodic, spontaneous, and/or random monitoring of treatment progress by the health care provider. An algorithm analyzes previous data captured by the medical monitoring device and determines whether the polling will be by continuous, periodic, spontaneous, and/or random monitoring. Through the polling feature, health care providers can monitor seriously ill patients outside the hospital, almost as if they were receiving bedside monitoring in the hospital. Moreover, in cases where the patient's treatment regimen includes an "unforgiving" medication that must be taken according to precise schedules and quantities to avoid dire clinical problems, the polling feature provides the health care provider with the ability to remotely, yet closely, monitor health status data and compliance with treatment regimens.

In appropriate cases (based on the particular requirements of the treatment regimen being administered), physiologic, cellular, molecular, endocrine and/or metabolic events can be assessed and monitored, and resulting data is transferred to the medical monitoring device from remote monitors 106 and/or sensors 107, as illustrated in Fig. 24; or the medical monitoring device itself assesses and monitors such events. The remote monitors 106 and sensors 107 can be linked to the medical monitoring device directly or by a wireless means, as illustrated in Fig. 24. The resulting data is either stored in the medical monitoring device for subsequent transmission (i.e., during a subsequent polling operation), or promptly transmitted to the remote device 100 for storage and analysis. As described above, the timing and content of the transmission of the resulting data depends on the events or data monitored, or information generated therefrom, the disease being treated and the treatment regimen being administered, and is set or established by a healthcare provider accordingly.

The medical monitoring device analyzes the resulting data by comparing it to target data. Then, if the device determines that the resulting data meets certain predefined criteria, the device promptly transmits the resulting data to the remote device 100 for storage and analysis by the healthcare provider. The predefined criteria are set or established by a healthcare provider, and depend upon the treatment being administered and the particular data being analyzed, as described above. The criteria may comprise target data from which the resulting data should not deviate in a given manner, a template with which the resulting data is compared, or any other method for analyzing the resulting data. Further, the resulting data that triggers a prompt transmission may comprise a single data element, a particular sequence of data elements, or a particular combination of data elements. The timing or immediacy of the transmission also depends on the disease being treated and the treatment regimen being administered, and is set by the healthcare provider accordingly. The events being assessed and monitored may include, but are not limited to, EEG, EKG, blood pressure, pulse, temperature, blood glucose level, and/or cellular, molecular, and endocrine or metabolic events, as specified above. The end result is the ability to analyze and correlate physiologic and physical data, medication compliance data, and health status assessment data, and develop a complete picture of the patient's health and treatment progress. Following the analysis, prompt transmission of new information, patient queries, sensor or monitoring device queries, or instructions then alter the treatment regimen in real-time with respect to monitored events.

The wireless communication feature provides improvements over the prior art in that a medical monitoring device with such a feature does not necessarily require a memory storage function. The wireless communication feature also facilitates continuous, periodic, spontaneous, and/or random data transmission or polling of data related to the patient's treatment regimen and health status. The wireless communication feature thereby enables a varying degree of frequency and intensity in monitoring a patient's health status and treatment progress, including variation in the types of data transmitted and monitored, and enables monitoring at a level that provides real-time interaction with outpatients. Further, the wireless communication feature provides for prompt data transfer where the patient's condition warrants prompt attention. The wireless communication feature thereby allows for outpatient treatment of seriously ill patients, to keep them out of hospitals and nursing homes, with more independence, and less health care expense.

Further, seriously ill outpatients can be treated by a team of professional that may include one or more physicians, a case manager, a visiting nurse, a physical therapist, a personal emergency response center, psychiatrist, etc. This team is analogous to a "code blue" team, a surgical team, or other type of healthcare team for inpatient care. While the device of the present invention monitors different aspects of the patient's condition, it may become necessary that one or more of the team members be promptly notified about emerging aspects of the patient's physical state. Then, according to the results of algorithms that analyze monitored information concerning the patient's physical state, the medical monitoring device determines which team members to notify, the timing of such notification, and the appropriate data to convey; and transmits the data accordingly. The notification consists of the transmission of information concerning the patient's condition, and occurs either by a wireless communication or through a network of modems 102, or other appropriate transmission method, as illustrated in Fig. 24.

For example, with regard to an outpatient with a congestive heart failure condition, when the device detects an increase in the patient's blood pressure of at least 30mm mercury, an algorithm could provide that the device notify the patient's case manager of the developing circumstances. Further, if the increase in blood pressure is accompanied by chest pain, then the algorithm could provide that the device notify the patient's case manager and visiting nurse. Going one step further, if certain cardiac enzyme elevations, muscle receptor alterations, or other molecular changes are occurring, the algorithm could provide that the device notify the case manager, visiting nurse, and cardiologist. Finally, if the patient also increases nitroglycerin tablet intake, the algorithm could provide that the device notify a nearby emergency response center and request the dispatch of an ambulance, in addition to notifying the above mentioned healthcare providers.

As part of the process of moving patient care from the hospital and doctor's office, into the home and ambulatory setting, we have previously described a patient monitoring system that includes a medication monitor that monitors medical treatment, medication use, patient health status, and quality of life; monitors that are external, or implanted, that monitor physiologic functions including but not limited to temperature, pulse, EKG, and PO2; and external or implanted monitors that monitor hormone levels, ions, cellular mechanisms and molecular events. Algorithms developed by healthcare professionals were described that would determine how, when, where, and with what degree of urgency the data from the monitors would be transmitted to the health caregivers.

Algorithms are provided that function like a physician conducting a clinical examination. Algorithms within the device determine when events should be monitored, which events to monitor, how those events should be monitored, and the particular monitors and monitoring sequence employed. As illustrated in Fig. 24, the monitoring is accomplished through a system of monitors 106 and sensors 107 interfaced with the medical monitoring device either by a direct connection, or by a wireless means. The algorithms then analyze the data collected, and determine the urgency of communicating the results of the analysis and coordinate such communications, as described above.

For example, in monitoring and treating the patient with congestive heart failure condition, a medication use monitor, clinical health status monitor, blood pressure monitor, pulse oximetry monitor, EKG monitor, implanted or external blood and serum monitors (to monitor physiologic and cellular events), and other external or implanted monitors, are applied to the patient. The various monitors interface with the medical monitoring device, which contains a series of algorithms for analyzing data collected from the monitors and coordinating the appropriate treatment and communications procedures. The monitored data, for example, may indicate an increased use of nitroglycerin tablets and increased blood pressure. An algorithm analyzes the data and activates the pulse oximetry monitor, which shows reduced oxygen in the blood. The analysis triggers another algorithm that activates the EKG monitor, and activates serum monitors to monitor for cardiac enzyme elevations indicative of heart muscle damage. A further algorithm analyzes the data from all the monitors and determines a heart attack is underway. The medical monitoring device then activates a medication discharge device that administers a drug to block the heart attack, and facilitate notification of all the pertinent healthcare providers and ambulance service. While waiting for the ambulance, the medical monitoring device continues to monitor the data from the monitors, transmit data to the healthcare providers, and continues the administration of additional drugs, and titrate the dosing of the drugs based upon monitored events that relate to blood levels of the drug, degree of heart muscle damage, degree of chest pain experienced by the patient, etc.

In addition to activating monitors singly, in sequence, or in combination based upon the analyzed data, the medical monitoring device may contain additional algorithms that regulate other functions. For example the algorithms could regulate the duration of monitoring the patient by one or more monitors- One such algorithm could determine and control the specific tests administered by a particular monitor.

The medical monitoring device itself, in addition to storing and executing the algorithms, contains the monitors for performing the necessary monitoring and testing for analyzing the patient's medication use, health status, quality of life, physiologic status, cellular function, hormone levels, receptor levels, ions, cell counts, etc.

Accordingly, the medical monitoring device continuously analyzes data coming in from certain monitors, and continuously alters the selection, combination, timing, duration, sequencing, and activation of monitors to most effectively monitor and treat the patient's condition in real-time. The medical monitoring device further controls the communication of information to the healthcare treatment team, to create a seamless and integrated monitoring and communication system. Through a series of patient monitors, software-driven algorithms, and communications linkages to healthcare professionals, the present invention provides a real-time patient event-monitoring system that automatically conducts clinical examinations that are continuously modified and refined based upon the patient's real-time clinical needs.

A medical monitoring method and apparatus is provided that carries out real-time monitoring of a patient's health status and automatic modification of a treatment regimen. The medical monitoring device monitors patient data relating to one or more physical attributes of the patient, and analyzes that data in accordance with an algorithm stored in the medical monitoring device- The medical monitoring device then provides instructions to the patient, or modifies the patient's treatment regimen, when the analysis reveals that the physical attributes meet certain predefined criteria. As described above, the predefined criteria are set or established by a healthcare provider, and depend on the disease(s) being treated and treatment regimen being administered.

For example, if the medical monitoring device monitors certain conditions or events, or receives response data, indicative of a congestive heart failure condition, then the device would instruct the patient to take certain medications, go to the emergency room, or modify the patients treatment regimen (i.e., medication schedule), accordingly. The algorithm is developed by a healthcare provider, and executes an analysis (i.e., decision tree analysis) that results in different actions depending on the monitored conditions or events.

Other anticipated medical conditions include diabetes, renal or kidney disease, liver disease, hypertension, endocrine imbalance, cancer, and lung and heart disease. Also included are viral, bacterial and fungal diseases, and antigens associated with specific human pathogens (i.e., if the patient is on antibiotics, a healthcare provider will monitor whether the organism becomes resistant and increases in number).

Functionality is provided like that of a nervous system or neuroregulatory system, paralleling, for example, the neuroendocrine system. In the human neuroendocrine system, sensors in the brain monitor a variety of physiologic and hormonal processes, and then activate glands to release varying levels and types of hormones and peptides to maintain homeostasis in the body. The system is linked together via neural pathways, and a feedback loop system. Common to many disease-states is a disruption of physiologic processes that serve to cause or worsen the underlying disease-state, whereby the effects of the disruption require correction as part of the disease treatment. A classic example is the hypothalamic-pituitary-thyroid axis, wherein the brain normally senses the amount of circulating thyroid hormone and, based upon the levels of thyroid sensed by the hypothalamus in the brain, increases or decreases the output of thyroid hormone by the thyroid gland. With a thyroid disease, however, the thyroid gland does not properly respond to the brain's regulatory signals, and outputs too much or too little hormone independent of the brain's commands.

A single medical monitoring device 5, or a system of monitoring devices 106, is provided that interfaces with systems of sensors 107 that may be external to or implanted within the patient. Different sensors are responsible for monitoring different processes in the body, and are designed to communicate with one another via wireless means, or by a direct connection. For example, one sensor might be responsible for monitoring circulating peptide, molecular, ionic, and hormone levels in the blood, and additional levels, such as glucose, potassium, and oxygen (pO2) levels. A second sensor might be responsible for monitoring heart function. A third sensor would monitor pulmonary (lung) function. A fourth sensor would monitor blood pressure. Additional sensors would monitor additional processes.

In addition, a variety of pharmaceutical, hormone, nutriceutical, and other supplementation delivery devices 108, linked with the system of sensors and the monitoring device(s), deliver appropriate types and quantities of pharmaceuticals, hormones, nutriceuticals, and other supplements based upon processes and levels monitored by the sensors. The medical monitoring devices include algorithms that control the release of the supplementary and pharmaceutical substances from the delivery devices, based on an analysis of the data collected by the system of sensors.

The algorithms determine and control the activation of particular sensors at particular times or during particular time intervals, and the communication between particular sensors, including the timing of such communications, the nature and type of communication, and the type of information communicated. The overall system is integrated and coordinated to monitor physiologic processes related to the patient's disease state(s). The monitors function like a "brain", controlling the monitoring processes and the delivery of supplements. They compare the monitored processes and levels to normative physiologic values, determined and preprogrammed into the monitors by healthcare professionals, and then effectuate a series of treatments to restore homeostasis. The algorithms are designed to function like the neuroregulatory systems of the brain, analyzing monitored data, comparing the monitored data to predetermined norms, and effectuating outputs in the form of treatments delivered to the patient, further diagnostic tests, alerts issued to healthcare professionals, and other remedial functions.

A central medical monitoring device 105 implanted within the patient, or remote from the patient, performs a polling function as described in detail above, whereby it periodically polls the system of sensors and monitors via a wireless means to obtain the monitored data. The central monitor 105 functions as command and control center, governing and regulating all of the above processes. The central monitor 105 also performs diagnostics on the other monitors 5 and 106, sensors 107, and delivery devices 108, and, if a malfunction is detected, the central monitor 105 analyzes the malfunction and either corrects it and/or notifies the appropriate healthcare professional. The present embodiment enables the replacement of defective systems of the body by a system of sensors and monitoring devices linked to a central medical monitoring device.

For example, a patient may be attached to a glucose monitor that monitors glucose levels in the blood and reports them to the central medical monitoring device via wireless transmission, or when the central device polls the glucose monitor's memory for stored levels of glucose. Upon receiving the data representing glucose levels, the central device executes an algorithm that compares the levels to normative values. When the glucose levels are within the range of normative values, the central monitor determines that no treatment is necessary. When the reported glucose levels are outside the range of normative values, the central device instructs the patient to administer an injection with additional insulin, or eat a piece of fruit or candy bar, depending upon whether the glucose levels are too high or too low, respectively. Alternatively, the central device activates an implanted insulin pump to release insulin into the patient's bloodstream, and/or contacts a healthcare professional that the patient is in danger.

Through this wireless system that maintains homeostasis, the patient's defective systems are replaced or supplemented by a wireless monitoring and therapeutic system, that establishes appropriate feedback loops and algorithms, to optimize health outcomes outside of a hospital setting.

The central monitor links up with one or more databases 109 for: (1) better analyzing the inputted data from the peripheral sensors and monitors; (2) downloading appropriate information from the databases and communicating that information to the patient; (3) applying database information to deploy and control other monitors; and (4) applying database information to deliver medical treatment and medication.

For example, a glucose monitor attached to a patient transmits data indicating the patient's glucose level to the central monitor, at selected time intervals and via a wireless means. The central monitor also periodically polls an insulin administration monitor to receive data indicating the date, time, and dosing amount of the patient's insulin medication. The central monitor then polls a diabetes disease management database to access and/or download algorithms that, when applied to analyze the glucose data levels and insulin dosing levels, provide new instructions for the patient about insulin dosing and glucose monitoring. Further, the central monitor accesses a nutrition database, downloads new dietary instructions, and communicates the new insulin instructions and dietary instructions to the patient.

When further monitoring reveals a glucose level that is dangerously high, posing a risk for the development of a hyperglycemic coma, the central monitor again polls the diabetes disease management database, and accesses algorithms that determine the need for cardiovascular monitoring. The central monitor then initiates blood pressure and cardiac status monitoring. The central monitor receives data concerning the patient's cardiac status, and again accesses the diabetes disease management database algorithms which determine that the next insulin dose should be increased by 50%. The central monitor communicates the new dosing instructions to the patient.

Alternatively, the peripheral system of monitors, sensors, and delivery devices are preferably equipped with the ability to access the databases themselves and directly communicate between one another, thereby bypassing the central monitor. This provides a backup system, should the central monitor fail, or should such an alternative be more desirable based on cost, portability, efficiency, or other factors.

By linking a variety of patient monitors and sensors with one another, and with various databases; and by providing a series of algorithms that analyze monitored data, combined with information from the databases; and by initiating various treatment functions (i.e., communicating treatment information to the patient or administering doses through attached medication delivery systems) based on the results of algorithm-based data analysis; real-time patient monitoring and management of health status is provided, independent of or in conjunction with the healthcare professional. The system also takes advantage of continuously updated databases and knowledge systems, that can be used to guide treatment of the patient's condition in real-time, to optimize the monitoring and management of disease.

A multitude of different algorithms may be developed that provide for prompt transmission of patient data should a single data element, sequence of data elements, or combination of data elements concerning the patient's health status be entered into or monitored by the medical monitoring device. It is therefore intended that the foregoing detailed description be regarded as illustrative rather than limiting, and that it be understood that the following claims define the scope of the present invention.

## Claims

1. A method for performing real-time monitoring of a patient's health status using a medical monitoring device capable of data transmission, said method comprising the steps of:
executing an interaction between the medical monitoring device and the patient, whereby the patient interacts with the medical monitoring device through dedicated keys and soft function keys, wherein the interaction generates resulting data, and the resulting data comprises data concerning adherence to a medical treatment regimen, the treatment regimen comprising the patient's scheduled medication times and doses;
analyzing the resulting data by comparing it to target data,
if the resulting data meets certain predefined criteria upon analysis, promptly transmitting at least a portion of the resulting data from the medical monitoring device to a remote device (100) when an execution command requires immediate transmission,
else, storing the resulting data in the medical monitoring device when the execution command does not require immediate transmission; and
automatically adjusting the medical treatment regimen based on the resulting data.

2. The method of claim 1, whereby the resulting data further comprises one or more types of data from a group consisting of response data provided during a query mode of the medical monitoring device, data concerning physical health, data concerning physiological status, data concerning cellular functions, data concerning molecular events, and data concerning endocrine and metabolic events, and
the method further comprising the step of comparing the response data with target data, and, when the response data meets certain predefined criteria, promptly transmitting at least a portion of the response data from the medical monitoring device to the remote device (100).

3. The method of claim 1 or 2, whereby the medical monitoring device performs transmission of data wirelessly.

## Patentansprüche

1. Verfahren zum Durchführen einer Echtzeit-Überwachung des Gesundheitszustandes eines Patienten unter Verwendung einer zur Datenübertragung fähigen medizinischen Überwachungsvorrichtung, wobei das Verfahren die folgenden Schritte umfasst:
Ausführen einer Interaktion bzw. Wechselwirkung zwischen der medizinischen Überwachungsvorrichtung und dem Patienten, wobei der Patient mit der medizinischen Überwachungsvorrichtung durch bestimmte Tasten und Softfunktionstasten interagiert, wobei die Interaktion sich ergebende Daten erzeugt und die sich ergebenden Daten Daten umfassen, welche sich auf die Einhaltung eines medizinischen Behandlungsplans beziehen, wobei der Behandlungsplan die zeitlich geplanten Arzneimittelanwendungszeiten und Dosierungen des Patienten umfassen;
Analysieren der sich ergebenden Daten, indem sie mit Zieldaten verglichen werden,
wenn die sich ergebenden Daten nach der Analyse gewisse vordefinierte Kriterien erfüllen, unverzügliches Übertragen wenigstens eines Teils der sich ergebenden Daten von der medizinischen Überwachungsvorrichtung an eine entfernte Vorrichtung (100), wenn ein Ausführungskommando bzw. Befehl die sofortige Übertragung erfordert,
ansonsten Speichern der sich ergebenden Daten in der medizinischen Überwachungsvorrichtung, wenn das Ausführungskommando keine sofortige Übertragung erfordert; und
automatisches Einstellen des medizinischen Behandlungsplans basierend auf den sich ergebenden Daten.

2. Verfahren nach Anspruch 1, wobei die sich ergebenden Daten ferner eine oder mehrere Arten von Daten aus einer Gruppe umfassen, die besteht aus: Antwortdaten, die während einer Abfragebetriebsart der medizinischen Überwachungsvorrichtung bereitgestellt werden, Daten, die sich auf die physische Gesundheit beziehen, Daten, die sich auf den physiologischen Zustand beziehen, Daten, die sich auf zellulare Funktionen beziehen, Daten, die sich auf molekulare Ereignisse beziehen, und Daten die sich auf endokrine und metabolische Ereignisse beziehen, und
das Verfahren ferner den Schritt des Vergleichens der Antwortdaten mit Zieldaten umfasst, und, wenn die Antwortdaten gewisse vordefinierte Kriterien erfüllen, wenigstens einen Teil der Antwortdaten unverzüglich von der medizinischen Überwachungsvorrichtung an die entfernte Vorrichtung (100) überträgt.

3. Verfahren nach Anspruch 1 oder 2, wobei die medizinische Überwachungsvorrichtung die Datenübertragung drahtlos durchführt.

## Revendications

1. Procédé pour accomplir en temps réel la surveillance de l'état de santé d'un patient en utilisant un dispositif de surveillance médical capable de transmettre des données, ledit procédé comprenant les étapes suivantes :
l'exécution d'une interaction entre le dispositif de surveillance médical et le patient, sachant que le patient interagit avec le dispositif de surveillance médical par l'intermédiaire de touches dédicacés et de touches de fonction, opération pendant laquelle l'interaction génère des données de résultat, et sachant que les données de résultat comprennent des données concernant la conformité à un régime de traitement médical, le régime de traitement comprenant les temps et les doses de médicaments élaborés pour le patient ;
l'analyse des données de résultat par une comparaison aux données cibles,
si les données de résultat sont conformes à un certain critère prédéfini suite à l'analyse, la transmission prompte d'au moins une partie des données de résultat depuis le dispositif de surveillance médical à un dispositif éloigné (100) lorsqu'une instruction d'exécution exige une transmission immédiate ;
autrement, la mémorisation des données de résultat dans le dispositif de surveillance médical lorsque l'instruction d'exécution n'exige pas une transmission immédiate ; et
la mise au point automatique du régime de traitement médical sur la base des données de résultat.

2. Procédé selon la revendication 1, dans lequel les données de résultat comprennent en outre un ou plusieurs types de données parmi un groupe constitué des données de réponse fournies pendant un mode d'interrogation du dispositif de surveillance médical, des données concernant la santé physique, des données concernant l'état physiologique, des données concernant les fonctions cellulaires, des données concernant les événements moléculaires, et des données concernant les événements de la glande endocrine et métaboliques, et
le procédé comprenant en outre l'étape de comparer les données de réponse aux données cibles, et, si les données de réponse sont conformes à un certain critère prédéfini, l'étape de transmettre promptement au moins une partie des données de réponse depuis le dispositif de surveillance médical au dispositif éloigné (100).

3. Procédé selon l'une ou l'autre des revendications 1 et 2, dans lequel le dispositif de surveillance médical accomplit la transmission des données sans câble.
